(19)

Europäisches
Patentamt
European
Patent Office
Office européen
des brevets

(11) **EP 1 895 436 A1**

(12) **EUROPEAN PATENT APPLICATION**

(43) Date of publication:
**05.03.2008 Bulletin 2008/10**

(51) Int Cl.:
***G06F 19/00*** (2006.01)

(21) Application number: **06018169.0**

(22) Date of filing: **31.08.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(71) Applicant: **Silicos NV
3590 Diepenbeek (BE)**

(72) Inventors:
• **De Winter, Hans Louis Jos
2970 Schilde (BE)**
• **Langenaeker, Wilfried Gert Roger
3720 Kortessem (BE)**

(74) Representative: **Bird, William Edward et al
Bird Goën & Co.
Klein Dalenstraat 42A
3020 Winksele (BE)**

(54) **Method for evolving molecules and computer program for implementing the same**

(57) A computer-based method and system of evolving a virtual molecule with a set of desired properties is described that begins with extracting fragments from existing molecules and labelling those fragments. Connectivity rules existing between the fragments in the existing molecules are determined followed by combining these fragments according to the connectivity rules. The molecules generated by the combination are evaluated and some are selected for modification. The evaluation and modification steps are repeated for the selected molecules until either 1) a target evaluation value is achieved or 2) the evaluation step has been performed a predefined number of times.

Figure 1

**Description**

Field of invention

The invention relates to the field of molecular modeling for drug discovery and to the application of genetic algorithms for chemical discovery and in particular to the use of computer based systems. In particular, a method is disclosed to generate novel virtual molecules based on genetic programming in combination with experimental data of the binding of real molecular species to a target protein. The present invention also includes a manufacturing method of molecules. Once obtained, selected virtual molecules are chemical synthesized.

Background of the invention

Virtual synthesis

The assembly of novel molecules by means of computer algorithms is not new. The first applications of such *de novo* approach can be found in the protein structure-based development of molecular structures with favorable binding affinity for the binding pocket of a protein. Caflisch and coworkers [Caflisch et al. (1993) J. Med. Chem. 36, 2142-2167] searched for molecular functional groups that fit in the binding pocket of a target protein and connected these groups with known scaffolds from a database. 'Legend' is a computer program written by Nishibata and coworkers [Nishibata et al. (1993) J. Med. Chem. 36, 2921-2928] to generate new molecules on an atom-by-atom basis using a set of definitions of allowed bond lengths and bond angles. A third approach is illustrated by the computer program 'SPROUT' [Gillet et al. (1994) J. Chem. Inf. Comput. Sci. 34, 207-217] in which in first instance functional moieties are searched to fit in the binding pocket of a target protein. In a second phase, new molecular structures are generated by connecting the functional moieties with molecular scaffolds. The difference with the work of Caflisch is that in this case novel scaffolds are generated from molecular fragments rather than retrieving existing scaffolds from a database.

Among the experimental approaches, great progress has been made in the domains of parallel synthesis and combinatorial libraries [Bradley (2004) Horizon Symposium Nature]. The initial ambition to explore the chemical space 'at full power', something in which millions of dollars have been invested, has been tempered quite quickly by the enormous scale of the chemical space, unforeseen problems by the actual synthesis and extremely disappointing results [Dickson & Gagnon (2004) Nat. Rev. Drug Discov. 3, 417-429; Service (2004) Science 303, 1796-1799]. The decision to introduce as much diversity as possible within the resulting combinatorial libraries often yielded molecules that were difficult to synthesize, unstable, or simply not interesting from a pharmaceutical point of view. For this reason libraries nowadays are getting more and more optimized towards specific problems, for example against the inhibitory effect towards well-defined protein target classes. Such an optimization is quantified by means of some kind of scoring mechanism. However, the exact balance between diversity and focus is still far from clear, but as a general rule one can state that the required degree of diversity is inversely related to the available knowledge of the pharmacological target [Hann & Green (1999) Curr. Opin. Chem. Biol. 3, 379-383]. In addition, not only the biological activity of a molecule is important, but also other parameters that transform a molecule into a medicine, such as toxicity, molecular weight, absorption and metabolism, have to be taken into account.

The development of virtual compound libraries is spurred by the observations that 1) the total number of molecules starting from all available fragment libraries is too large to be synthesized, and 2) more intelligent technologies are required to generate pharmacologically interesting lead compounds. In general, two approaches exist to generate novel virtual molecular libraries. The first is reagent-based. This approach is conceptually very close to the synthesis as performed in the chemical laboratory, whereby reagents are combined in a computational manner using a number of chemical rules [Lobanov & Agrafiotis (2002) Comb. Chem. High Throughput Screen. 5, 167-178]. However, in general these methods are quite slow [Leach & Hann (2000) Drug Discov. Today 5, 326-336].

The second method is fragment-based. Following this approach one starts with a molecular scaffold (Markush structure) of which the R-groups are substituted by relevant monomers from fragment libraries [Leland et al. (1997) J. Chem. Inf. Comput. Sci. 37, 62-70; Agrafiotis (2002) J. Comput. Aided Mol. Des. 16, 335-356].

Related to the fragment-based approach is the development of combinatorial schemes. In this approach, a large number of components are selected with which all possible combinations may be generated using combinatorial chemistry. This approach often leads to a better final diversity, but requires improved optimization- or sampling schemes [Agrafiotis & Lobanov (2000) J. Chem. Inf. Comput. Sci. 40, 1030-1038; Leach & Hann (2000) Drug Discov. Today 5, 326-336].

The selection of building blocks (reagents or fragments) for the generation of a virtual library of molecules is a crucial step and depends on a number of objectives such as 1) the required diversity, 2) the required affinity with the target protein, and 3) a combination of 1) and 2). It is therefore important, when discussing the *de novo* molecular design or virtual synthesis, to select an appropriate scheme for representing molecules. The way molecules are represented by a computer algorithm has a direct impact on the required methodology to generate novel molecules *in silico.* Visual representations are not suitable for processing by computer programs. Three-dimensional representations such as tables with coordinates and distance matrices are also too complex to be useful in virtual synthesis.

A first type of molecular representation that is used in the virtual synthesis is a string-based representation. A common notation in this class is known as the SMILES notation [Weininger (1988) J. Chem. Inf. Comput. Sci. 28, 31-36]. New molecules are generated by defining and applying operators to manipulate these strings [Kamphausen et al. (2002) J. Comput. Aided Mol. Des. 16, 551-567; Venkatasubramanian et al. (1995) J. Chem. Inf. Comput. Sci. 35, 188-195].

The most powerful molecular representation from an algorithmic point of view is the notation as a graph or tree structure ['Computational Medicinal Chemistry for Drug Discovery', edited by Bultinck et al., published by Marcel Dekker, USA]. Different levels of implementation can be distinguished. Starting from the molecular connectivity table, graphs may be constructed whereby the individual atoms are represented by the nodes and the bonds by the edges of the graph. At a higher level, certain configurations may be combined into fragments that form as a whole a structure, for example a benzene ring. This fragment-based representation is an important component of several algorithms [Brown et al. (2004) J. Chem. Inf. Comput. Sci. 44, 1079-1087; Globus et al. (1999) Nanotechnology 10, 290-299; Nachbar (2000) Genetic Programming and Evolvable Machines 1, 57-94]. Bemis and Murcko [Bemis & Murcko (1996) J. Med. Chem. 39, 2887-2893] demonstrated the classification of a large number of known medicines with a limited number of rings, linkers, and side chains.

Scoring functions

Within a virtual synthesis context, the quality of the generated virtual molecules can be measured by means of appropriate scoring functions. With emphasis on drug discovery applications, scoring functions can be classified in two major groups. First, if the three-dimensional structure of the target protein is known, then the fit between the generated virtual molecule and the potential protein binding pocket can be used as scoring measure. This approach is generally defined as the protein structure-based scoring approach. Examples of well-known docking algorithms include DOCK [Ewing et al. (2001) J. Comput. Aided Mol. Des. 15, 411-428], FLEXX [Rarey et al. (1996) J. Mol. Biol. 261, 470-489], GLIDE [Halgren et al. (2004) J. Med. Chem. 47, 1750-1759], and GOLD [Jones et al. (1997) J. Mol. Biol. 267, 727-748]. A nice review of scoring functions is provided by Perola and coworkers [Perola et al. (2004) Proteins 56, 235-249].

Secondly, if the structure of the target protein is not known but information of medicines that bind to the target does exist, then the similarity between the generated virtual molecule and the known drug can be used as scoring function. This is generally termed ligand-based scoring and a number of approaches have been described: molecular similarities calculated from topology-based fingerprints [Barnard (1993) J. Chem. Inf. Comput. Sci. 33, 532-538], alignment of three-dimensional structures [Klebe et al. (1999) J. Comput. Aided Mol. Des. 13, 35-49; Lemmen & Lengauer (2000) J. Comput. Aided Mol. Des. 14, 215-232; Grant et al. (1996) J. Comp. Chem. 17, 1653-1666], three-dimensional pharmacophore matching [Sheridan et al. (1989) Proc. Natl. Acad. Sci. USA. 86, 8165-8169].

Optimization

The generation of small, strongly focused libraries of molecules is a trend in the world of chemo-informatics [Gillet (2004) Methods Mol. Biol. 275, 335-354; Valler & Green (2000) Drug Discov. Today 5, 286-293]. The main idea behind this approach is to generate molecules that are as specific as possible against a given pharmacological target, and are obtained by integrating this pharmacological knowledge with the virtual synthesis by means of the above-mentioned scoring functions in combination with appropriate optimization algorithms. This process of *de novo* design can be translated as a non-analytical optimization problem. Recently a number of publications have appeared in which different optimization algorithms have been evaluated within the context of the virtual synthesis process.

Genetic algorithms of genetic programming are the most widely used methods to generate virtual molecules according to specific target functions. Genetic programming is a specific adaptation of the genetic algorithm in which the chromosomes are represented as graphs or tree structures instead the standard binary or number-based representations. Venkatasubramanian [Venkatasubramanian et al. (1995) J. Chem. Inf. Comput. Sci. 35, 188-195] was one of the first to apply a genetic algorithm based on string notations for the generation of polymers from a set of fragments. A similar approach was later introduced by Kamphausen [Kamphausen et al. (2002) J. Comput. Aided Mol. Des. 16, 551-567]. In patent US 5,434,796, Weininger and coworkers describe a SMILES-based genetic algorithm method to generate molecular libraries according specific target functions. Nachbar encodes the topology of molecules as tree structures [Nachbar (2000) Genetic Programming and Evolvable Machines 1, 57-94]. Globus and coworkers also use a tree structure to represent molecules, and describe a number of crossover operators between pairs of molecules [Globus et al. (1999) Nanotechnology 10, 290-299]. Finally, Brown *et al.* [Brown et al. (2004) J. Chem. Inf. Comput. Sci. 44, 1079-1087] have described a genetic algorithm that is based on the ideas of Globus, but focused on the generation of 'average' molecules that represent, to a certain level, the characteristics of a number of known molecules. For this purpose, the similarity with these molecules was selected as a scoring function.

The use of alternative optimization algorithms has also been described extensively in the literature. Zheng presented a simulated annealing procedure based on a special protocol for multitarget optimization [Zheng (2004) Methods Mol. Biol. 275, 379-398]. Young and coworkers implemented an alternating algorithm for the generation of both focused as diverse libraries [Young et al. (2003) J. Chem. Inf. Comput. Sci. 43, 1916-1921]. Scoring functions were the similarity with a given target molecule or a given structure-activity relationship. Miller and coworkers [Miller et al. (2003) J. Chem. Inf. Comput introduced an approach based on information theory. Sci. 43, 47-54]. Their approach allows inclusion of different molecular properties simultaneously. Schneider and Nettekoven propose a slightly different strategy as a scoring function [Schneider & Nettekoven (2003) J. Comb. Chem. 5, 233-237]. Firstly, a self-organizing map (SOM) was trained based on the binding of known molecules with a specified protein target. Secondly, this SOM was used as a scoring tool of the generated virtual molecules.

A number of concepts for the generation of *de novo* molecules can also be applied in virtual combinatorial chemistry. Agrafiotis [Agrafiotis (2002) J. Comput. Aided Mol. Des. 16, 335-356] describes the implementation of a simulated annealing procedure and evolutionary algorithm for the generation of virtual combinatorial libraries. Gillet and coworkers introduced the program 'SELECT', a genetic algorithm for the development of combinatorial libraries whereby a weighed sum of different scoring functions is used as target function [Gillet et al. (1999) J. Chem. Inf. Comput. Sci. 39, 169-177]. In subsequent work, 'MoSELECT' was introduced and allowed the simultaneous optimization of multiple target functions without having to calculate a weighed sum from all individual objectives [Gillet et al. (2002) J. Chem. Inf. Comput. Sci. 42, 375-385].

A practical example of virtual synthesis by using a genetic algorithm is disclosed in patent US 5,434,796. In this prior art, molecules represented as SMILES strings are evolved by mutations and selections of muted molecules in function of their fitness values. Those values are evaluated by a fitness function serving as the selection pressure. This prior art patent uses a string based representation of molecules which has the inconvenience of limited incorporation of the intrinsic chemical knowledge and chemical content. As a consequence, there is a real danger of arriving at results which are not relevant from a chemical point of view, unless additional software algorithms are implemented to overcome this issue.

There is therefore a need in the art for an improved virtual synthesis method which provides a new powerful molecular description tool allowing a higher degree of chemical accuracy. There is also a need in the art for an improved virtual synthesis method wherein an improved synergy between experimentally available data and molecular evolution algorithm leads faster to more potentially active compounds.

<u>Summary</u>

The present invention has the object to provide methods and apparatus for molecular modeling, e.g. for drug discovery, for molecular discovery and in particular to the use of computer based systems. In particular, the present invention provides methods and apparatus for generating novel virtual molecules based on genetic programming in combination with experimental data on the binding of real molecular species to a target molecule such as a protein. The target protein can be a synthetic or natural protein. A further aspect of the present invention is to use these novel virtual molecules to lead to the chemical synthesis of such molecules. The method may make use of a computer or a computing system. The invention results from the unexpected finding that the use of a two-level representation of virtual molecular fragments, including a level where atoms are labeled by labels giving information relative to both the chemical nature and the connectivity of the atoms, permits use of very generic operators at a high abstraction level easily implemented, e.g. in an object-oriented programming environment. A method of evolving a virtual molecule with a set of desired properties according to the present invention involves a number of steps. The first step consists in storing *in silico* labelled fragments of existing molecules in one or more machine readable fragment databases, said labelled fragments having one or more open connections. The labelled fragments are obtainable by, for example,

    (i) labelling with labels chosen atoms of a set of represented existing molecules, each label giving information relative to both the chemical nature and the connectivity of said atoms,

    (ii) determining which label is connected to which label in each of said represented existing molecules and storing this information as connectivity rules in a connectivity database, and

    (iii) cutting said represented existing molecules into one or more labelled fragments.

Connectivity represents here the ability of the labelled atoms to form covalent bonds.

The second step consists in generating one or more virtual molecules by combining at least two of the labelled fragments from the one or more fragments database by matching the labels according to the connectivity rules from the connectivity database.

The third step consists in determining the degree of fitness of each virtual molecules against a fitness or goal function or functions.

The fourth step consists in selecting one or more times at least one virtual molecule correlating positively with the degree of fitness.

The fifth step consists in modifying each of the at least one selected virtual molecule.

The sixth step consists in repeating iteratively the process going from the third step to the fifth step until either:

1) the degree of fitness of at least one of the virtual molecules selected during the fourth step is equal or higher than a predefined target degree of fitness or

2) the fourth step has been performed a predefined number of times.

When (1) or (2) is achieved, the fifth step is no longer performed and the seventh step is performed instead.

The seventh step consists in generating a data file comprising electronic data representing at least one virtual molecule selected during the sixth step. The electronic data include information relating to physical characteristics of the molecule.

The present invention should speed-up the process of drug design, discovery and identification. Application of the invention can be found in the discovery of novel lead compounds for the treatment of human and veterinary diseases, as well as in the domains of plant and material protection.

For any of the embodiments of the present invention a step may be included of synthesising a molecule based on the molecular modelling according to the present invention, e.g. on selected molecules designed in accordance with the methods of the present invention. Accordingly, the present invention includes a method of manufacturing a molecule comprising the steps of:

a) using a computer for evolving a virtual molecule with a set of desired properties followed by synthesising the molecule, the evolving method including storing *in silico* labelled fragments of represented existing molecules in one or more fragment databases, said labelled fragments having one or more open connections, said labelled fragments being obtainable by:

(i) *in silico* labelling chosen atoms of a set of represented existing molecules with labels, each label giving information relative to both the chemical nature and the connectivity of said atoms, and
(ii) cutting said represented existing molecules into one or more labelled fragments,

b) determining which label is connected to which label in each of said represented existing molecules and storing this information as connectivity rules in a connectivity database,
c) generating one or more virtual molecules by combining at least two of said labelled fragments from said one or more fragments database by matching said labels according to said connectivity rules from said connectivity database,
d) determining the degree of fitness of each said virtual molecules against a fitness function,
e) selecting at least one virtual molecule correlating positively with the degree of fitness,
f) modifying each of said at least one selected virtual molecule,
g) repeating iteratively steps (d) to (f) until either:

1) the degree of fitness of at least one of said virtual molecules selected in (e) is equal or higher than a predefined target degree of fitness or
2) step (e) has been performed a predefined number of times,

wherein once (1) or (2) is achieved, step (h) is performed instead of step (f),
h) generating a data file comprising electronic data representing the at least one virtual molecule selected in (g)
i) synthesising at least one of the molecule selected in step g).

The present invention also provides a computer-based system for evolving a virtual molecule with a set of desired properties including:

- means for storing *in silico* labelled fragments of existing molecules in one or more fragment databases, said labelled fragments having one or more open connections, said labelled fragments being obtainable via:

(i) means for labelling chosen atoms of a set of represented existing molecules with labels, each label giving information relative to both the chemical nature and the connectivity of said atoms,

(ii) means for determining which label is connected to which label in each of said represented existing molecules and storing this information as connectivity rules in a connectivity database, and
(iii) means for cutting said represented existing molecules into one or more labelled fragments,

- means for determining which label is connected to which label in each of said represented existing molecules and storing this information as connectivity rules in a connectivity database,
- means for generating one or more virtual molecules by combining at least two of said labelled fragments from said one or more fragments database by matching said labels according to said connectivity rules from said connectivity database,
- means for determining the degree of fitness of each said virtual molecules against a fitness function,
- means for selecting at least one virtual molecule correlating positively with the degree of fitness,
- means for modifying each of said at least one selected virtual molecule,
- means for repeating iteratively steps (c) to (e) until either:

1) the degree of fitness of at least one of said virtual molecules selected in (d) is equal or higher than a predefined target degree of fitness or
2) step (d) has been performed a predefined number of times,

wherein once (1) or (2) is achieved, step (g) is performed instead of step (e)

- means for generating a data file comprising electronic data representing the at least one virtual molecule obtained in (g).

For any of the apparatus embodiments of the present invention, apparatus may be included for outputting a representation of a molecule in sufficient detail for the molecule to be synthesised. Examples of the output are string or token representations (for example the SMILES representation), connectivity tables such as, but not limited to, the structure-data format of MDL. For any of the apparatus embodiments of the present invention, apparatus may be included for synthesizing a molecule based on the molecular modeling according to the present invention.

The present invention includes computer program products such as software for implementing any of the methods of the invention. For example, the present invention also includes a machine-readable data or signal carrier storing an executable program which implements any of the methods of the present invention when executed on a computing device. Such a data carrier may be a magnetic storage device such as a diskette, hard driven magnetic tape or an optical data carrier such as a DVD or CD-ROM, solid state memory such as a USB memory stick, flash memory, etc.

Brief description of the figures

Figure 1 is a flowchart showing a process to design novel virtual molecules according to an embodiment of the present invention.

Figure 2 is a flowchart showing a process to obtain labeled fragments and connectivity rules during the analysis phase according to an embodiment of the present invention.

Figure 3 is a schematic representation describing the atom labeling step during the analysis phase according to an embodiment of the present invention.

Figure 4 is a schematic view of an example molecule on which fragments types are identified according to an embodiment of the present invention.

Figure 5 is a schematic representation describing the storage of labeled fragments and connectivity rules into databases according to an embodiment of the present invention.

Figure 6 is a flowchart showing the virtual synthesis phase using genetic programming according to an embodiment of the present invention.

Figure 7 is a schematic representation describing a de novo synthesis step according to an embodiment of the present invention.

Figure 8 is a schematic representation describing the process of side-chain mutation according to an embodiment of the present invention.

Figure 9 is a schematic representation describing the cross-over process according to an embodiment of the present invention.

Figure 10 is a flowchart showing a way to obtain weight factors according to an embodiment of the present invention.

Figure 11 is an example of a computer system that may be used with the present invention.

Figure 12 is a schematic representation of the cutting process according to an embodiment of the present invention.

Figure 13 represents the chemical structures of Nutlin-2 and the corresponding modified version that has been used as a reference molecule in example 2.

Figure 14 represents the best molecule from the genetic algorithm population after 1,000 cycli and the reference molecule from example 2.

Detailed description of the invention

The present invention will be described with reference to certain drawings and to certain embodiments but this description is by way of example only.
In a first embodiment, the present invention relates to a computer-based method of evolving or manufacturing a virtual molecule with a set of desired properties for binding at a protein target or any other suitable target comprising the steps of:

a) storing *in silico* labelled fragments of represented existing molecules in one or more fragment databases, the one or more fragment databases being machine readable by a computer system, the labelled fragments having one or more open connections and being obtainable by:

(i) *in silico* labelling chosen atoms of a set of represented existing molecules with labels, each label giving information relative to both the chemical nature and the connectivity of the atoms, and
(ii) cutting the represented existing molecules into one or more labelled fragments,

b) determining which label is connected to which label in each of the represented existing molecules and storing this information as connectivity rules in a connectivity database,
c) generating one or more virtual molecules by combining at least two of the labelled fragments from the one or more fragments database by matching the labels according to the connectivity rules from the connectivity database,
d) determining the degree of fitness of each of the virtual molecules against a fitness function,
e) selecting at least one virtual molecule correlating positively with the degree of fitness,
f) modifying each of the at least one selected virtual molecule,
g) repeating iteratively steps (d) to (f) until either:

1) the degree of fitness of at least one of the virtual molecules selected in (e) is equal or higher than a predefined target degree of fitness or
2) step (e) has been performed a predefined number of times, Wherein once (1) or (2) is achieved, step (h) is performed instead of step (f),

h) generating a data file comprising electronic data representing said at least one virtual molecule selected in (g).

The target protein may be a natural or synthetic protein. Other suitable target molecules are for instance any molecule capable of eliciting an immune reaction from a mammal such as a human, i.e. any molecule or structure that contains an immugenic determinant. For example, such a target molecule may have a pocket or molecular feature to which an antibody can bind.
As an optional feature, the data file further comprises electronic data representing the degree of fitness of the at least one virtual molecule or another value correlating with the degree of fitness. This is advantageous because it provides to the user information whether the molecule is worth being synthesised or not.
As an optional feature, the other value referred to in the paragraph hereabove is a predicted biological activity.

As an optional feature, the predicted biological activity referred to in the paragraph hereabove is a binding affinity to the protein target.

As an optional feature, the labelled fragments having one open connection may be stored into a first fragment database and the labelled fragments having two or more open connections may be stored in a second fragment database. This optional feature is advantageous because it leads to a significant speed up of the fragment retrieval process.

As an optional feature, the number of virtual molecules generated in step (c) may be from 50 to 1000. This is advantageous because it allows for the generation of a broad family of molecules with diverse properties. In addition, keeping the number of virtual molecules between 50 to 1000 enables the process to be easily parallelised by distributing each of the different molecules to separate CPU's.

As an optional feature, the at least two labelled fragments selected in step (c) may be selected randomly in the one or more fragment database. This is advantageous because it ensures a high probability of generating novel molecules.

As an optional feature, each of the labelled fragments may be associated to a weight factor and, in step (c), the at least two labelled fragments may be selected correlating positively with said weight factor. This is advantageous because it speeds up the convergence of the synthesis procedure and guarantees the virtual synthesis procedure to be guided into a particular direction of the chemical space.

As an optional feature, chosen atoms may be all atoms but hydrogen atoms. This is advantageous because this speeds up the labelling process.

As an optional feature, in step (c) and in the case where each of the labelled fragments is associated to a weight factor, the weight factor may correlate positively with an experimentally determined binding affinity between real molecular species and the protein target or other target molecule, wherein the real molecular species are structurally related to the labelled fragment to which the weight factor is associated. This is advantageous because it speeds up the convergence of the synthesis procedure toward virtual molecules having high affinity for the protein target or other molecule.

As an optional feature, the real molecular species may have a molecular weight smaller or equal to 350 g/mol. This is advantageous because they are the molecules the most likely to show high structural similarity with the labelled fragments used for the virtual synthesis.

As an optional feature, the binding affinity may be determined via one of the following technique: X-ray crystallography, NMR, mass spectrometry, microcalorimetry, solid-phase detection, in vitro binding assay, sedimentation analysis or capillary electrophoresis.

As an optional feature, the binding affinity may be binary, i.e. qualitative, i.e. not existing or existing. This is advantageous because it usually permits to gather data over a large range of real molecular species much faster than when quantitative binding affinity data are retrieved. The treatment of binary data is also faster so that the overall process is speeded up.

As an optional feature, in step (c) and in the case where each of the labelled fragments is associated to a weight factor, the weight factor may correlates positively with the frequency of its associated labelled fragment in the one or more fragment databases. This is advantageous because it leads to a more likely selection of fragments that are more common in the ensemble of represented existing molecules, and therefore more likely in term of synthetic accessibility.

As an optional feature, the existing molecules may be stable under normal physiological conditions. This is advantageous because it raises the chances for the virtual molecules generated by the virtual synthesis process of the present invention to be, once chemically synthesised, stable under normal physiological conditions and therefore usable in medical or pharmaceutical applications.

In a second embodiment, the present invention relates to a computer program product comprising software code for implementing any of the above embodiment and features when executed on a computing system.

In a further embodiment, the present invention relates to a machine readable data carrier storing the computer program of the embodiment above.

In an embodiment, the present invention relates to a computer based method of evolving a virtual molecule with a set of desired properties.

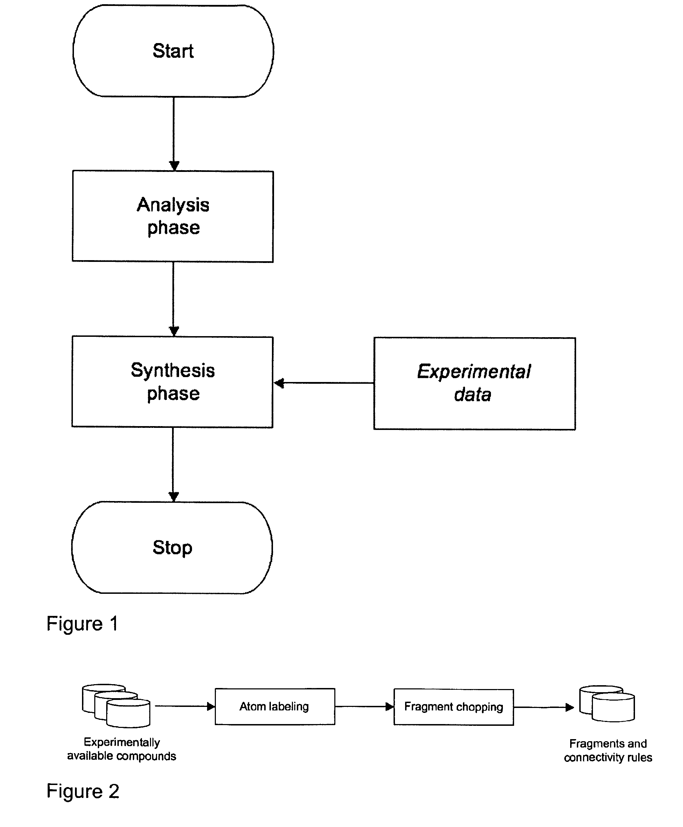

The overall flow of the method according to this first embodiment of the present invention is shown in Figure 1 and involves the following phases:

1) In the first phase, later referred as the analysis phase, chemical knowledge is extracted from existing molecules. During this phase, information is acquired regarding the topology and bond connectivities in existing molecules, and this knowledge is stored in a number of appropriate databases;

2) In the second phase, later referred as the synthesis phase, the acquired knowledge from the 'analysis' phase is combined to generate new virtual molecules that are optimised towards a user-defined set of desired properties using a genetic programming approach. In order to speed up the optimisation or to guide the optimisation into a specific area of the chemical space, weight factors are optionally included which may be, for instance, derived from experimentally-determined binding data.

The set of desired properties is defined by the user as a target degree of fitness. A virtual molecule will be considered as possessing the user defined set of desired properties when its degree of fitness calculated against a fitness function at least equals a target degree of fitness. The fitness function aims at evaluating if a virtual molecule would have, once chemically synthesised, a particular biological activity such as but not limited to the binding affinity to a protein target or other target and, potentially, a pharmaceutical effect or a therapeutic effect. The analysis phase will now be described. A flow chart of the different steps of the analysis phase is provided in Figure 2. Referring to Figure 2, the first step of the analysis phase comprises the collection of a large number of representations of existing molecules, i.e. representation of commercially or experimentally available compounds known to be stable under normal physiological conditions. By representation of a molecule, it is meant a one, two or three-dimensional representation *in silico* of a molecule. Once these represented existing molecules have been collected and stored into databases, the second step of the analysis phase comprises an atom labelling procedure based on an appropriate labelling scheme. Third, each of the labelled compounds are then chopped, i.e. cut into a set of predefined fragments such as side-chains and linkers, while the original connecting bonds between the different fragments are translated into a set of connectivity rules. This translation step can be either anterior, posterior or simultaneous to the cutting step. Finally, the generated fragments and connectivity rules are stored in databases according to specific formats for easy retrieval during the synthesis phase.

The first step of the analysis phase, consisting in collecting representations of molecules known to be stable under normal physiological conditions, can be performed by collecting them from a number of publicly available (for example the NCI) or commercially available libraries of existing molecules. In order to improve the relevance of the constituting molecules, an optional cleaning procedure may be included to filter out the molecules which are not 'drug-like' or which are composed of undesired fragments such as for example nitro functionalities. For instance, an appropriate cleaning step can be performed using the computer program 'Filter v2.0' [OpenEye Scientific Software, Santa Fe, USA]. Preferably, molecules matching at least one of the following rules are removed from the library of existing molecules:

o Molecules containing an atom which is different from the following: H, C, N, O, F, S, Cl, Br, I, Si, B, P.
o Molecules containing a functional group which is one of the following:

quinone; pentafluorophenyl esters; paranitrophenyl esters; triflates; lawesson-s-reagent; phosphoramides; acylhydrazide; cation C, Cl, I, P, or S; phosphoryl; alkyl phosphate; phosphinic acid; phosphanes; phosphoranes; chloramidines; nitroso; N-, P-, S-halides; carbodiimide; isonitrile; triacyloxime; cyanohydrins; acylcyanides; sulfonylnitrile; phosphonylnitrile; azocyanamides; beta-azocarbonyl; polyenes; saponin derivatives; acid halide; aldehyde; alkylhalide; anhydride; azide; azo; dipeptide; michael acceptor; betahalocarbonyl; nitro; oxygen cation; peroxide; phosphonic acid; phosphonic ester; phosphoric acid; phosphoric ester; sulfonic acid; sulfonic ester; tricarbophosphene; epoxide; sulfonylhalide; halopyrimidine; perhalo-ketone; aziridine; alphahalo-amine; halo-amine; halo-alkene; acyclic NCN; acyclic NS; $SCN_2$; terminal vinyl; hydrazine; N-methoyl; NS-betahalothyl; propiolactones; nitroso; iodoso; iodoxy; N-oxide; iodine; phosphonamide; alphahalo ketone; oxaziridine; sulfonimine; sulfinimine; phosphoryl; sulfinylthio; disulfide; enol ether; enamine; organometallic; dithioacetal; isothiocyanate; isocyanate; carbamic acid; triazine; nonacylhydrazone; thiourea; hemiketal; hemiacetal; ketal; aminal; hemi-aminal; benzyloxycarbonyl; tert-buthoxycarbonyl; fluorenylmethoxycarbonyl; trimethylsilyl; tert-butyldimethylsilyl; triisopropylsilyl; tert-butyldiphenylsilyl.

o In the case of a salt with two or more individual chemical components, the smallest fragment (for example, the anorganic counterion such as Na+ or Ca2+, or the organic counterion such as maleate).

Other exclusion rules than the two rules cited above are of course applicable, function of the user-defined set of desired properties.

The next step of the analysis phase, consists in labelling chosen atoms of the set of represented existing molecules obtained in the previous step with labels, each labels giving information relative to both the chemical nature and the connectivity of the atoms. Preferably, all atoms but hydrogen atoms are labelled.

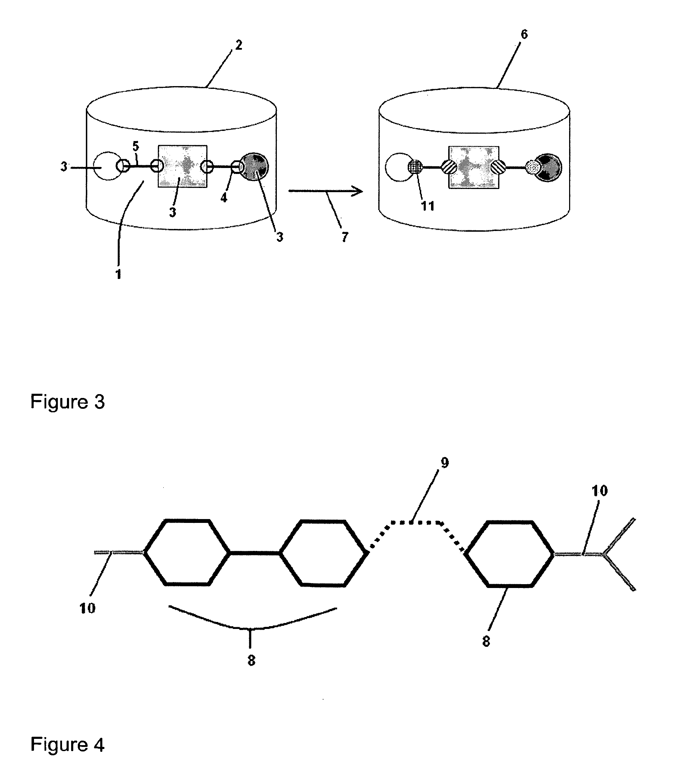

Figure 3 provides a schematic illustration of the labelling process. At the left side of Figure 3, a database of existing molecules (2) is shown. Within this database (2), an existing molecule (1) is schematically represented which comprises constitutive fragments (3) including atoms (4) and bonds (5). The differences in grey-scale indicate differences in atomic constitution. At the right side of figure 3, a database of labelled existing molecules (6) labelled with labels (11) resulting from an atom labelling step (7) is represented. The same existing molecule (1) is schematically represented which now has its atoms labelled. Labels are differentiated by the hashing used.

The labelling procedure can be implemented in different ways, but has consequences for the subsequent synthesis steps. A very simple labelling system, which does not represent the direct atomic environment of each of the chosen atoms, will lead to a larger diversity in the subsequent synthesis steps but at the expense that many of the resulting virtual molecules might contain chemically unstable and irrelevant bonds. On the other hand, a very complex labelling procedure in which the direct atomic environment of each of the chosen atoms is represented in great detail, will lead to a limited but nevertheless chemically relevant set of virtual molecules. Different choices of labelling procedure leads to different balances between the resulting molecular diversity and the chemical relevance of generated virtual molecules. For instance, the labelling procedure was based on the original MMFF-94 force field [Halgren (1996) J. Comp. Chem. 17, 490-519; Halgren (1996) J. Comp. Chem. 17, 520-552; Halgren (1996) J. Comp. Chem. 17, 553-586; Halgren (1996) J. Comp. Chem. 17, 616-641. Halgren (1999) J. Comp. Chem. 20, 720-729; Halgren (1999) J. Comp. Chem. 20, 730-748; Halgren & Nachbar (1996) J. Comp. Chem. 17, 587-615]. Preferably, the labels are those of table 1 below but other labelling strategies may be implemented as well.

Table 1

| Label | Definition |
|---|---|
| 0 | None of the atoms below |
| 1 | >C< |
| 2 | >C=R |
| 3 | >C=X (with X = O, P, N, S) |
| 4 | =C= *or* -C#R |
| 5 | C in aromatic ring |
| 6 | R-C(=R)-R{1} |
| 7 | >C=N {1} |
| 8 | >N- |
| 9 | -N=N or -N=C |
| 10 | >N-C=X (with X = O, S) or >N-N=R |
| 11 | >N{1}< |
| 12 | >N-C=R or >N-C#C |
| 13 | >N-C#N *or* N-S(=O) |
| 14 | >N{1}=O |
| 15 | >N {1}=R |
| 16 | -N{1}#C |
| 17 | -N{-1}-R |
| 18 | N{1} in aromatic ring |
| 19 | N in aromatic ring |
| 20 | -O- |
| 21 | -O{1}- |
| 22 | P with 3 bonds |
| 23 | P with 4 bonds |
| 24 | -S- |
| 25 | >S= |
| 26 | S with 4 bonds or S(=R)(=R)=R |
| 27 | -S(=R)-R{-1} |
| 28 | =S=O |
| 30 | -Cl |

(continued)

| Label | Definition |
|---|---|
| 31 | -Br |
| 32 | -F |
| 33 | -1 |
| 34 | =O |
| 35 | =S |
| 36 | >N-aromatic |
| 37 | -O{-1 } |
| 38 | -O-C=X (with X = O, S) |
| Wherein '-' represents a single bond, '=' a double bond, '>' and '<' two single bonds, '#' a triple bond, {n} a charge with absolute value equal to n, and 'R' an alkyl group. | |

A next step of the analysis phase consists in cutting the labeled represented existing molecules into one or more labeled fragments having one or more open connections and storing these labeled fragments into one or more fragment databases. The fragments are composed of rings, linkers, and side-chains (see Figure 4).

Figure 4 shows a represented existing molecule composed of two ring systems (8), a linker (9) and two side-chains (10).

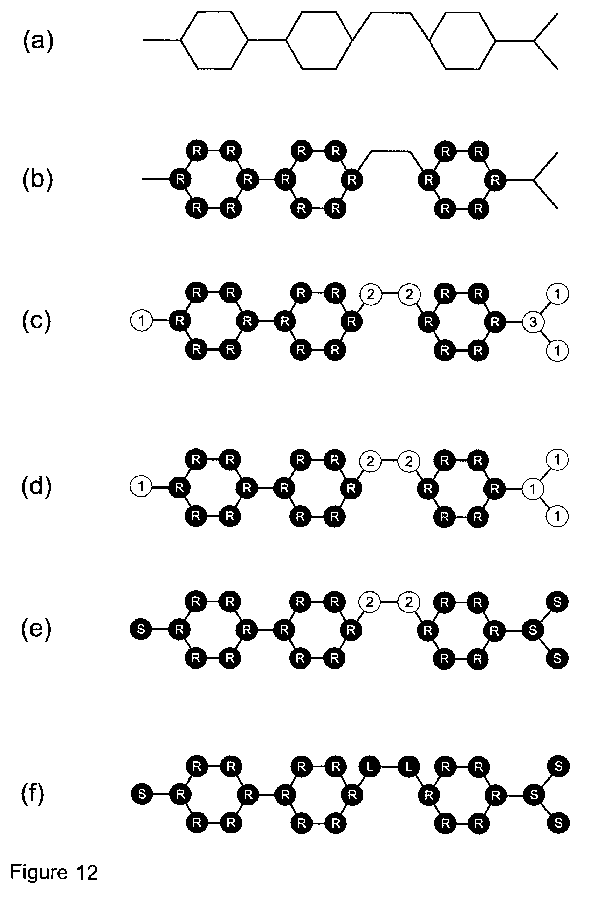

The entire cutting process is performed in three steps as illustrated in Figure 12:

(Figures 12a to 12b) In a first step, atoms which are part of a ring system are identified and labelled as such. A number of ring perception algorithms have been published, including the work of Balducci and Pearlman [Balducci & Pearlman (1994) J. Chem. Inf. Comput. Sci. 34, 822-831] and implemented in the OEChem C/C++ library of OpenEye Scientific Software (Santa Fe, USA).

In a second step, the 'connectivities' of the remaining non-ring atoms is determined. In this context, 'connectivity' is defined as the number of neighbouring atoms with more than one 'connectivity'. This process performed in a cyclic manner:

(Figures 12b to 12c) An initial 'connectivity' is attributed to each non-ring atom as being the total number of connected atoms.
(Figures 12c to 12d) The 'connectivities' are refined by setting the updated 'connectivity' to the number of connected atoms that have a 'connectivity' larger than one.
The Step of Figures 12c to 12d is repeated until no changes in the refined 'connectivities' occur.
(Figures 12d to 12e) All atoms with a final 'connectivity' of one are labelled as being side-chain atoms.
(Figure 12e to 12f) In a final step, linkers are defined from the set of all remaining atoms as those atoms that have a final 'connectivity' higher than one.

Following the chopping process, all fragments are grouped together according their number of 'open connections'. An 'open connection' is defined as an atom in the fragment that was originally bonded to an atom of another fragment in the parent molecule, but where the bond has been removed during the chopping process. All fragments with only a single 'open connection' are treated as 'side-chains', while all fragments with more than one 'open connection' are treated as 'linkers'. The original classification of linkers, side-chains, and ring systems is therefore reduced during this step to solely linkers and side-chains, whereby the actual distinction is based on the number of 'open connections' that the particular fragment contains.

A next step of the analysis phase consists in determining which label is connected to which label in each of said represented existing molecules and storing this information as connectivity rules in a connectivity database.

Alternatively, if this step is performed after the cutting step, this step may consists in determining which label was connected to which label in each of said parent represented existing molecules and storing this information as connectivity rules in a connectivity database.

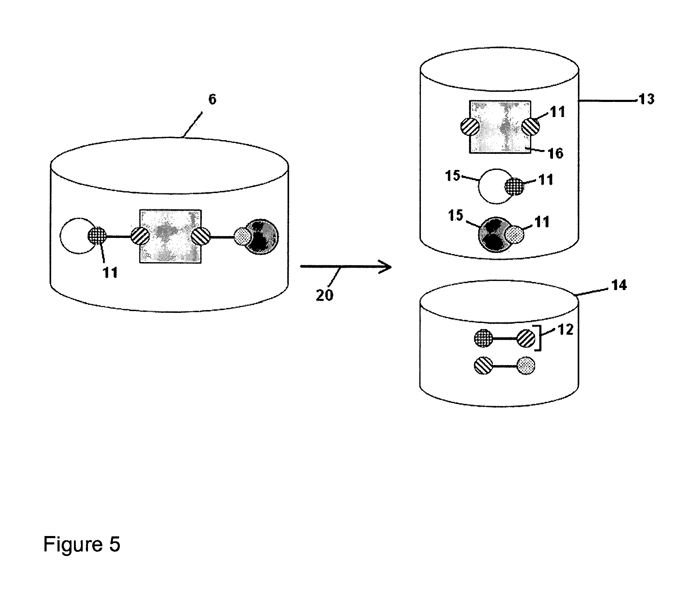

The storage of the extracted fragments and connectivity rules into fragment and connectivity databases respectively is schematised in Figure 5. On the left side of Figure 5, a database of labelled existing molecules (6) is represented. A labelled existing molecule is schematically represented within this database of labelled existing molecules (6). On the right side of Figure 5, two databases (13 and 14) resulting from the cutting process (20) are displayed. The database (13) displayed at the top on the right side of Figure 5 is a fragment database (13) containing fragments more precisely defined as linkers (16) and side-chains (15). The number of labels (11) present on each fragment is equivalent to the number of open connection possessed by this fragment. The database displayed at the bottom on the right side of Figure 5 is a connectivity database (14) which contains connectivity rules (12).

Database systems that can be used include but are not limited to the mySQL system. The actual choice of the database is not crucial to the performance of the described method.

Preferably, the fragments and connectivity rules are stored in three databases:

- A database containing all side-chains, in which a side-chain is defined as being a fragment with maximal one open connection.
- A database containing all linkers, in which a linker is defined as being a fragment with at least two open connections.
- A database containing all connectivity rules, in which a connectivity rule is defined as being the rule describing the atom labels that can be connected to each other.

A non-limitative example of format in which these fragments and connectivities can be stored in the database is based on a modified version of the SMILES language [Weininger (1988) J. Chem. Inf. Comput. Sci. 28, 31-36]. The particular suggested modification of the original SMILES involves the introduction of additional tokens to define the 'open connections' within each particular fragment, and is based on the use of '<' and '>' tags in combination with the atom label symbol of the original atom and with bond order information. For example, a phenyl ring with one 'open connection' would be stored in the database as c1cc (<5>) ccc1. In this example, the third carbon of the phenyl ring is of type '5' and was originally connected to another atom within the parent molecule via a single bond. The atom type to which this type '5' carbon was connected is stored in the connectivity database. A bond connection can also be of type double, like in NC (<=3>) N. In this example, the central carbon of ureum is of type '3' and was originally connected to another atom within the parent molecule via a double bond. The type of this other atom is not stored together with the fragment information, but is stored in the connectivity database.

As outlined before, in the synthesis phase, a genetic programming algorithm is implemented to generate novel virtual molecules that meet a set of desired properties. The genetic algorithm and the genetic operators must be specifically adapted to the two-level representation of molecules resulting from the labelling step and the cutting step. The two levels are as follow:

- at the first level, the virtual molecules are represented as a combination of two types of building blocks, namely side-chains with one labelled open connection and linkers with two or more labelled open connections. Within this abstraction, molecules are described as a superstructure of building blocks, which are the labelled side-chains and the labelled linkers.
- At the second and more detailed level, the superstructure is filled in as a set of molecular fragments consisting of the actual atoms. This atomic representation can for instance be achieved by means of SMILES strings [Weininger (1988) J. Chem. Inf. Comput. Sci. 28, 31-36], although other molecular representations are also possible.

The virtual synthesis process therefore consists in replacing each particular superstructure with the actual molecular fragments out of a fragment database. All steps of the manipulation process are performed at the first level of representation, with the sole exception that the determination of the degree of fitness of the virtual molecule is performed at the second level of representation. An advantage of the two-level representation is that it serves as a topological backbone during the virtual synthesis procedure. A particular sequence of building blocks (e.g. side-chain - linker - side-chain) can be defined in first instance, after which the actual molecular representation can be refined by filling in the building blocks with molecular fragments. Such an approach is easily implemented in an object-oriented programming environment, and has the additional advantage that very generic operators can be implemented at a high abstraction level.

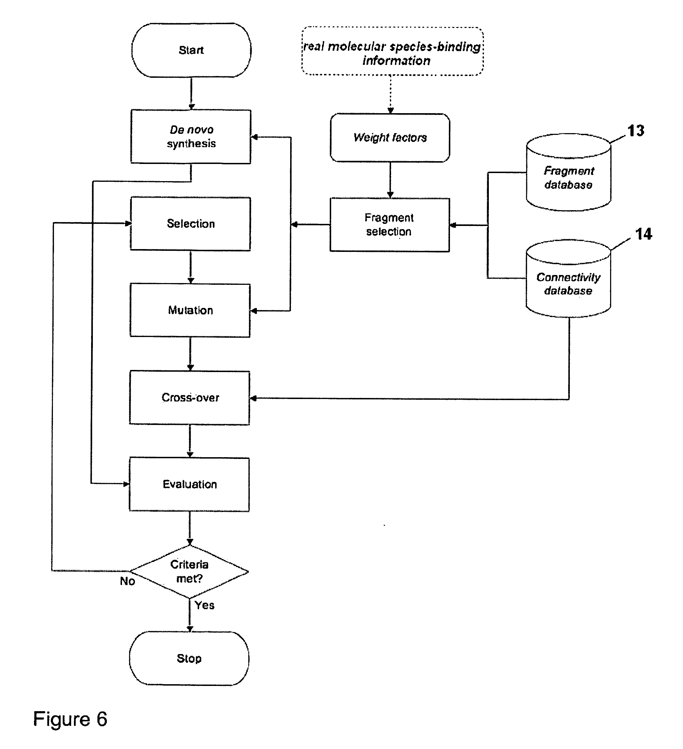

The overall flow of the synthesis phase is provided in Figure 6. The process starts by initiating the genetic population with a number of virtual molecules. It can be any number of virtual molecules but it is preferably a number comprised between 10 and 10000, most preferably between 50 and 1000 virtual molecules. These molecules are created by means of a *de novo* synthesis step, in which the virtual molecules are created by carefully selecting fragments and connectivity rules, and combining those fragments and connectivity rules into a new molecule. After initialization of the genetic population, in the evaluation step, the degree of fitness of each virtual molecule is determined against a fitness function. If one of the virtual molecules possesses a degree of fitness higher or equal to a predefined degree of fitness , the process stops. If the predefined degree of fitness is not achieved, by any of those virtual molecules, at least one virtual molecule is selected during the selection step with a probability of selection correlating positively with the degree of fitness of said at least one virtual molecule. In the next step, each selected molecule will be modified through a mutation step and/or a cross over step. New topologies are created during the modification step. From there on, the evaluation steps, the selection step and the modification step are repeated iteratively until the degree of fitness of at least one virtual molecule is equal or higher than the predefined target degree of fitness or until a predefined number of iterations is achieved.

The selection of new fragments from the fragment database that will be used in the *de novo* synthesis and mutation operations can be performed either as a strict random selection, or it can be implemented as a weighed selection in which the corresponding weight factors are for instance derived from:

- The relative occurrence of each fragment in the original molecular database of represented existing molecules.
- A random selection according to a normal distribution.
- Experimentally determined binding information of each fragment on the protein target or other target of interest. This list is not exhaustive and other ways to derive weight factors may be used.

Each of the different steps of the virtual synthesis phase is explained in more details in the following sections.

De novo synthesis

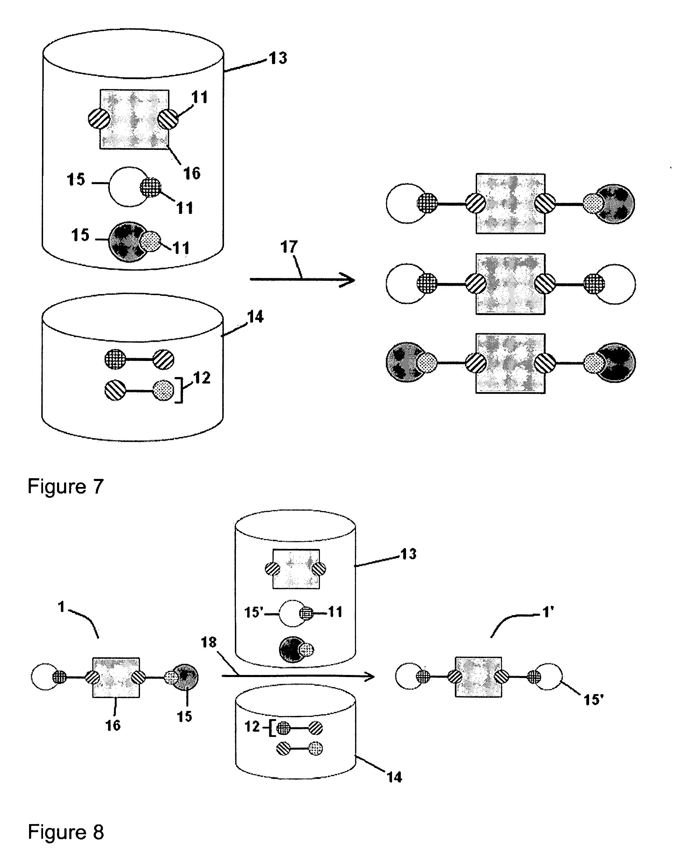

The initial *de novo* generation of new virtual molecules within the genetic population is based on the combination of fragments from the fragment database according to specific rules as defined by the connectivity rules from the connectivity database. A general overview of the process is schematically visualised in Figure 7.

On the left of Figure 7, a fragment database (13) containing side-chains (15) and linkers (16) is represented as well as a connectivity database (14) containing connectivity rules (12). On the right side of Figure 7, new virtual molecules are generated via the *de novo* synthesis step (17) by choosing and combining labelled fragments from the fragment database (13) and by matching labels (11) according to the connectivity rules (12). The *de novo* synthesis follows the following steps:

1) The total number of linker fragments (16) L of which the final molecule should consist is specified by the user. Preferably L is from 1 to 5.Typical values vary between 1 and 5.
2) An initial linker fragment (16) is selected from the fragment database (13) according to specific selection criteria.
3) If the number of linker fragments (16) within the virtual molecule is equal to L, the procedure continues at step 6.
4) A labelled open connection is randomly selected from the virtual molecule and a compatible linker fragment (16) is selected from the fragment database (13) according specific selection criteria. Two fragments are said to be compatible when the bond connecting these fragments is defined by the connectivity rules (12) within the connectivity database (14).
5) Go to step 3.
6) Fill up all remaining open connectivities with side-chain fragments according specific selection criteria.

Evaluation

All the virtual molecules within the genetic population are evaluated as whether the virtual molecule has a degree of fitness equal or higher than a predefined degree of fitness. This evaluation uses one or more fitness functions, which compare each virtual molecule to a given set of properties and provide a numerical measure of the degree of fitness of the virtual molecule to the set of properties. A plurality of fitness functions may be used to evaluate each of the virtual molecules within the genetic population. The multiple numerical scores can be combined into a single score by a mathematical transformation like for example addition or multiplication.

The user-defined fitness functions can be evaluated internally or externally. Preferably, they are evaluated externally to allow a high degree of flexibility. By internally, it is meant the here described computer program. By externally, it is meant one or more separate computer programs which are called from within the here described computer program. Since the two-level representation of virtual molecules within the method according to an embodiment of the present invention is different from the language that is used by existing programs that evaluate fitness functions, a conversion between these different representations is required. The majority of scoring programs are capable of processing molecular information in the form of SDF- [MDL Information Systems, Inc., USA] or SMILES-format [Weininger (1988) J. Chem. Inf. Comput. Sci. 28, 31-36], One or both of those formats can therefore be implemented to interchange virtual molecules and their structures with an external process or storage. For this, the second level of description of the virtual molecules is converted into those formats.

Evaluating the fitness functions externally increases the flexibility of the computer architecture used. For example, different scoring functions may be run on different computer systems, while yet another computer may be used to perform the database queries and virtual synthesis procedures. If the employed fitness function is very complex, as may be the case in for example quantum chemical calculations, a separate computer may be used for each molecule within the genetic population, whereby a set of computers may be run in parallel.

External programs may be used to create automatically three-dimensional coordinates from the second level of description. Examples of such programs include CORINA [Molecular Networks GmbH, Germany], CONCORD [Tripos Inc, USA], and OMEGA [OpenEye Scientific Software, USA]. A combination of these programs may be used to generate high-quality multiconformations of the virtual molecules. For example, the initial 3D-structure as generated by CORINA may be used as input to OMEGA to generate multiconformations of each virtual molecule.

Another advantage of evaluating the fitness functions externally is that many commercially available scoring programs may be incorporated within the flow of the present invention. Many external computational chemistry programs are available to evaluate these molecular fitness functions:

- Shape-based scoring programs include but are not limited to the program ROCS from OpenEye Scientific Software, USA [Grant et al. (1996), J. Comp. Chem. 17, 1653-1666]. ROCS matches the shape and chemical functionalities between a reference ligand and each of the population molecules, and returns for each molecule a shape similarity score as a number between 0 and 1 inclusive.
- Protein-based scoring programs include for example the programs DOCK [Ewing et al. (2001) J. Comput. Aided Mol. Des. 15, 411-428], FLEXX [Rarey et al. (1996) J. Mol. Biol. 261, 470-489], GLIDE [Halgren et al. (2004) J. Med. Chem. 47, 1750-1759], AUTODOCK [Morris et al. (1998), J. Comp. Chem. 19, 1639-1662], and GOLD [Jones et al. (1997) J. Mol. Biol. 267, 727-748]. For a complete review of methods see the publication of Perola and coworkers [Perola et al. (2004) Proteins 56, 235-249]. All these programs are designed to predict how small molecules, such as the virtual molecules within the genetic population, bind to a protein receptor of known 3D structure. When integrated in the genetic algorithm process of the present invention, all the docking programs try to fit each of the virtual molecules in the active site cavity of the protein of interest, and return a number to quantify the quality of fitting.
- Pharmacophore-based screening programs include but are not limited to CATALYST [Accelrys Software Inc., USA] and UNITY [Tripos Inc, USA]. The traditional medicinal chemistry definition of a pharmacophore is the minimum functionality a molecule has to contain in order to exhibit activity. In the pharmacophore-based screening programs, the fit between a reference pharmacophore and the pharmacophore generated from each of the virtual molecules is calculated and returned as a number.
- Topology-based scoring may be performed by calculating the similarity between the topology-based fingerprints of the reference compound and each of the virtual molecules. Similarity measures exist in many flavors, but the Tanimoto similarity coefficient is the most commonly used. This Tanimoto measure is a number between 0 and 1. A common program to generate topology-based fingerprints is the DAYLIGHT TOOLKIT from Daylight Chemical Information Systems, Inc..
- Field-based scoring is performed by calculating the similarity between the property fields surrounding a reference molecule and each of the virtual molecules. This can be done using Cresset's FIELDSCREEN program or with the Spectrophore™ technology from Silicos [Belgium]. In the case of the Silicos' Spectrophore™ technology, property fields may be calculated from any user-defined atomic property, although electrostatic charges, softness, hardness, electrophilicity, and lipophilicity are the most common used properties. The field-based similarity is calculated and returned in the form of a quantitative number.

The entire process to score the population molecules by means of external programs is summarized as follows:

1. Convert the internal two-layered molecular representation to a standard format like SDF- or SMILES, and write the virtual molecules in this format to a temporary file.

2. Convert the one-dimensional structures of the temporary file of step 1 into a set three-dimensional conformations, and write these structures to a second temporary file.

3. Evaluate each of the structures of the second temporary file of step 2 using one or more scoring functions, and write the scoring results into a third temporary file.

4. Read the scoring calculated functions and convert these using the appropriate mathematical transformations into a single fitness value. Use this fitness value to guide the selection.

Selection

Following the generation of the fitness values during the evaluation stage, the selection operator selects virtual molecules from the population for modification. The better the corresponding molecular fitness value, the higher the likelihood of being selected. Selection is done with replacement, meaning that the same virtual molecule can be selected more than once to become a parent.

Various selection schemes can be implemented. One example of selection scheme is the roulette-wheel selection, also called stochastic sampling with replacement [Baker (1987) Proceedings of the Second International Conference on Genetic Algorithms and their Application, Hillsdale, New Jersey, USA: Lawrence Erlbaum Associates, pp. 14-21]. This is a stochastic algorithm and involves the individual virtual molecules to be mapped to contiguous segments of a line, such that each molecule's segment is proportional in size to its fitness. A random number is generated and the molecule whose segment spans the random number is selected. The process is repeated until the desired number of individual virtual molecules is obtained. The stochastic method statistically results in the expected number of offspring for each virtual molecule. However, when the population is relatively small, the actual number of offspring allocated to a virtual molecule is often far from its expected value. An alternative method proposed by Baker is therefore a stochastic universal sampling method [Baker (1987) 'Proceedings of the Second International Conference on Genetic Algorithms and their Application', Hillsdale, New Jersey, USA: Lawrence Erlbaum Associates, pp. 14-21] to minimize spread and to provide zero bias. The virtual molecules are mapped to contiguous segments of a line, such that each molecule's segment is proportional in size to its fitness exactly as in roulette-wheel selection. Equally spaced selection pointers are placed over the line as many as there are molecules to be selected.

In order to keep the selection pressure relatively constant over the course of the virtual synthesis run, scaling methods may be used to map the 'raw' molecular fitness values to values that are less susceptible to premature convergence. One such approach is the 'sigma scaling', where each molecular fitness is transformed into an expected value which is a function of the molecular fitness, the population mean, and the population standard deviation [Goldberg (1989) 'Genetic algorithms in search, optimisation, and machine learning', Addison-Wesley].

Mutation

Once the selection operator has generated a new molecular population, the mutation operator transforms the topology of some of the virtual molecules. The total number of virtual molecules that are mutated is determined by the mutation probability, which varies between 0 and 100%, preferably between 0 and 50%. Preferably, the selection of molecules, which are to be mutated according this mutation probability, occurs entirely randomly.

Two particular types of mutations can be distinguished: sidechain mutations and linker mutations. In both cases, the mutation operator does not change the top-level structure of the molecules, i.e. each particular combination of sidechain and linker building blocks remains unaltered by the mutation operator.

Side-chain mutation

The replacement of one of the side-chains of a particular molecule by an other side-chain is a process termed side-chain mutation. This process is depicted in Figure 8.

On the left of Figure 8, a virtual molecule (1) is mutated (18) by exchanging a side-chain (15) by another side-chain (15') selected from a fragment database (13) as having a label compatible with the linker (16) according to the connectivity rules (12). On the right side of Figure 8, the mutated virtual molecule (1') bearing the new side-chain (15') is represented.

To ensure a correct behaviour of the mutation process, the allowed connectivity of the replacement fragment should be compatible with the original fragment connectivity.

The side-chain mutation process involves the following steps:

1. Randomly select a side-chain.
2. Select a compatible side-chain fragment from the fragment database according specific selection criteria (see section on fragment selection below). The new fragment is said to be compatible with the original fragment when the new fragment has a labelled open connection that can be connected to the labelled open connection of the virtual molecule according the rules as defined in the connectivity database. If a compatible fragment cannot be found in the database, the entire procedure is repeated from step 1 on. If still unsuccessful after a number of trials, then the procedure stops. The number of trials can be any number. Preferably, the number of trials is comprised between 1 and 1000, most preferably between 5 and 50.

Linker mutation

The replacement of one of the linkers within a particular molecule by an other linker is a process called linker mutation. This process is conceptually quite identical to the side-chain mutation process, with the exception that the number of 'open connections' may be different between the original linker and the new linker. These differences in number of 'open connections' will lead to changes in the overall topology of the resulting molecules, but not in the number of linker fragments:

1. Randomly select a linker and determine the number of linkers and side-chains that are connected to the selected linker fragment. The number of connected linkers is called $L$, and the number of connected side-chains is called $S$.

2. Randomly select the number of 'open connections' for the new linker ($N$). $N$ should be between 2 and the maximum number of 'open connections' in the linker database.

3. If $N$ is less than $L$, then $N$ is set equal to $L$ and all side-chains that are connected to the selected linker fragment are removed from the molecule. Continue at step 7.

4. If $N$ is equal to the sum of $S$ and $L$, continue at step 7.

5. If $N$ is higher than the sum of $S$ and $L$, continue at step 7.

6. If $N$ is smaller than the sum of $S$ and $L$, remove a randomly selected side-chain from the molecule and repeat this until the sum of $S$ and $L$ becomes equal to $N$. Continue at step 7.

7. Select a compatible linker fragment from the fragment database according to specific selection criteria (see section on fragment selection below). The new fragment is said to be compatible with the original fragment when the new fragment has labelled open connections which can be connected to the labelled open connections of the molecule according the rules as defined in the connectivity database. If a compatible fragment cannot be found in the database, the entire procedure is repeated from step 1 on. If still unsuccessful after a number of trials, the procedure stops. The number of trials can be any number. Preferably, the number of trials is comprised between 1 and 1000, most preferably between 5 and 50.

8. Fill up the remaining 'open connections' of the molecule with compatible side-chain fragments from the fragment database according specific selection criteria (see section on fragment selection below) and the rules as defined in the connectivity database.

Cross-over

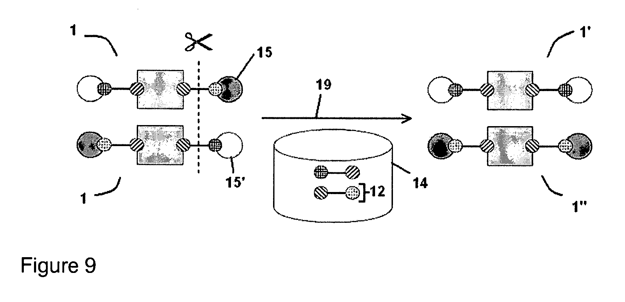

Another approach to create novel virtual molecules is by applying a cross-over (or recombination) operation between pairs of virtual molecules, whereby certain fragments between the two virtual molecules of the pair are interchanged (Figure 9).

On the left side of Figure 9, two virtual molecules (1), in this example identical, are crossed-over (19) by exchanging side-chains (15) and (15') by following the connectivity rules (12) to lead to two new virtual molecules (1') and (1") represented on the right side of Figure 9.

The total number of molecules that are submitted to the cross-over operator is determined by the cross-over probability, which can vary between 0 and 100%, preferably between 0 and 50%. The selection of virtual molecules that will be modified according this probability occurs preferably randomly.

Cross-over is a process in which relatively large modifications may be introduced within the superstructure of the virtual molecules. An important step in the cross-over process is the selection of the cross-over point in both parent virtual molecules. The cross-over point is located at the connection between two fragments in the superstructure of the respective virtual molecule. A complication hereby is that only connections of the same type, or compatible types, may be selected in both molecules:

1. Compare all labelled bonds of the first virtual molecule with all labelled bonds of the second virtual molecule. Randomly select a pair of labelled bonds that are compatible in terms of their connectivity rules.

2. If a compatible pair of labelled bonds has been found in step 1, reshuffle both virtual molecules by interchanging all connected linker and sidechain fragments.

By labelled bond, it is meant an entity "labelled atom - chemical bond - labelled atom".

Fragment selection

The *de novo* synthesis operator and the two mutation operators need to communicate with the fragment database for the selection of appropriate side-chain and linker fragments. This communication is implemented through a fragment selection operator. The main function of this operator is to retrieve the appropriate fragments from the fragment database, thereby taking into account the correct connectivity rules which are stored in the connectivity database.

The fragment selection process can be guided by a weight factor, whereby the fragments with higher weight will be more likely to be selected. To achieve this, the above mentioned roulette-wheel selection procedure can for instance be implemented.

For instance, weight factors can be derived from experimentally-determined knowledge regarding the affinity of certain chemical molecules, or fragments, to a particular protein target or other target molecule. The use of weight factor has the advantage of speeding up the convergence of the synthesis procedure and guarantees the virtual synthesis procedure to be guided into a particular direction of the chemical space. Examples of processes to obtain weight factors are described in the following section.

Incorporating molecular species-binding information

The process of incorporating molecular species-binding data into the synthesis phase is performed in three steps:

- Collection of fragment-binding data by means of experiments or by literature surveys which relates experiments;
- Converting the collected fragment-binding data into weight factors;
- Transforming of the weight factors by including other measures.

These three steps are exemplified in the following paragraphs.

Collection of fragment-binding data

Information regarding the affinity of fragments to protein targets or other target molecules can be derived by measuring experimentally the affinity of real molecular species to protein targets or other target molecules. A real molecular species is defined here as an organic molecule with a molecular weight smaller or equal to 350 g/mol.

The binding information can be obtained from a variety of sources. A good overview of the different approaches is given in the book 'Fragment-based approaches in drug discovery' [Jahnke, Erlanson, Mannhold, Kubinyi & Folkers (2006), published by Wiley] and also the review of Rees and coworkers [Rees et al. (2004) Nature Rev. Drug Discov. 3, 660-672]. In short, experimental methods to determine binding of real molecular species to proteins include but are not limited to:

- Protein X-ray crystallography [Hartshorn et al. (2005) J. Med. Chem. 48, 403-413]. Efficient fragment screening using protein X-ray crystallography requires the soaking of cocktails of real molecular species into pre-formed crystals of a target protein. After collection of the X-ray data, the identification of the active real molecular species from the cocktail is reliant on manual or automated analysis of the resultant electron density. The outcome of these studies is information regarding which real molecular species do bind to the protein target and the actual binding configuration in the active site. No information is obtained on the actual binding strength or affinity. The binding affinity that can be retrieved from this method will therefore be binary, e.g. have a value of either 0 or 1.
- NMR-based screening [Shuker et al. (1996) Science 274, 1531-1534], or Structure-Activity-Relationship (SAR) by NMR, involves identifying and interpretation of the chemical shifts in the NMR spectrum as a result of the binding of real molecular species to a target protein of interest. The result is information regarding the real molecular species that bind to the protein target. Typically, no information is obtained of the actual binding strength or affinity. The binding affinity that can be retrieved from this method will therefore be binary, e.g. have a value of either 0 or 1.
- The use of disulfide bonds to stabilize the binding of a real molecular species to the target protein [DeLano (2002) Curr. Opin. Struct. Biol. 12, 14-20]. This is achieved by placing a sulfur-containing amino acid called a cysteine on the surface of the protein and to screen the protein against a collection of sulfur-containing real molecular species. Real molecular species that bind near the cysteine form disulfide bonds with the protein, increasing the weight of the protein and allow the detection of the real molecular species by mass spectrometry. The outcome is a list of real molecular species that bind to the protein active site. No particular information is obtained regarding the real molecular species binding strength. The binding affinity that can be retrieved from this method will therefore be binary, e.g. have a value of either 0 or 1.
- Mass spectroscopy as a real molecular species-screening tool has been applied to RNA targets using high resolution Fourier Transform mass spectrometry [Swayze et al. (2002) J. Med. Chem. 45, 3816-3819]. In such a set-up, each real molecular species and target RNA is identified by its exact molecular mass. The identity of the real molecular species, the corresponding binding affinity, and the location of the binding site on the RNA can be determined in one set of experiments.
- Microcalorimetry-based real molecular species screening has been described in an application note of MicroCal LLC (USA) ['Divided we fall? Studying low affinity real molecular species of ligands by ITS', MicroCal LLC, USA, 2005] in which the heat generated by the real molecular species-protein binding process is measured and converted in thermodynamical parameters such as entropy and enthalpy measures. The outcomes of the experiments are the identities of the binding real molecular species and optionally the corresponding binding affinities.
- In-vitro binding assays which have been adapted to measure the binding of low affinity real molecular species have been described as well [Boehm et al. (2000) J. Med. Chem. 43, 2664-2674]. The results of these experiments are a set of real molecular species with their corresponding binding affinities for a particular protein target.
- Sedimentation analysis is a novel technology that has been described to measure real molecular species / protein interactions [Lebowitz et al. (2002) Protein Sci. 11, 2067-2079]. Sedimentation equilibrium measures the concentrations of the components at equilibrium in solution, and the readout from an sedimentation equilibrium experiments is an absorbance versus distance curve. The outcomes of the experiments are the identities of the real molecular species that show binding affinity for a particular protein target.

- Solid-phase detection is a general term covering a wide range of technologies that share a common working principle in which both a bioreceptor and a signal transducer are combined to detect the binding of real molecular species to proteins. The best known solid-phase detection method is surface plasmon resonance (SPR), which has originally been described and implemented by Graffinity Pharmaceuticals GmbH (Germany). The process involves a highly parallel production of chemical microarrays using proprietary, highly defined surface chemistry, followed by the simultaneous detection of protein interactions to 10,000 real molecular species via SPR imaging. Interaction data are combined with physicochemical compound data to interpret the array results. Alternative solid-phase detection methods include but are not limited to the rupture event scanning (REVS) and resonant acoustic profiling (RAP) technologies commercialized by Akubio Ltd (UK), reflectance interference (RIf), total internal reflection fluorescence (TIRF), and the microcantelever technology as commercialized by Concentris GmbH (Suisse).

- Capillary electrophoresis has also been mentioned as a tool to measure real molecular species / protein interaction [Carbeck et al. (1998) Acc. Chem. Res. 31, 343-350].

Complementary to the experimental approaches mentioned above to generate real molecular species-binding data, information gathered from literature sources may also be useful in generating knowledge about the affinity of certain fragments to specific protein targets or other target molecules.

Whatever approach is used to collect the real molecular species-binding data, the result is always a list of real molecular species structures which are known, or believed, to bind to the protein target or other target molecules. Quantitative affinity information in the form of dissociation or $IC_{50}$ values is useful, but not required. In its most strict form, only a binary 'yes' or a 'no' answer ('yes' indicates binding at certain protein and real molecular species concentrations, and 'no' indicates absence of interaction in the same conditions) is sufficient for the calculation of experimentally-based weight factors.

Conversion of the binding data into weight factors

The integration of all available real molecular species binding information into the synthesis phase is achieved by generating the appropriate weight factors from the binding information. However, for a number of reasons this conversion process is not straightforward: 1) the virtual fragments stored within the fragment database are different from the real molecular species, since the database fragments are purely virtual structures with a number of open connections; 2) in most cases, only qualitative binding information is available since quantitative affinity data are often difficult to obtain in a high-throughput experimental set-up.

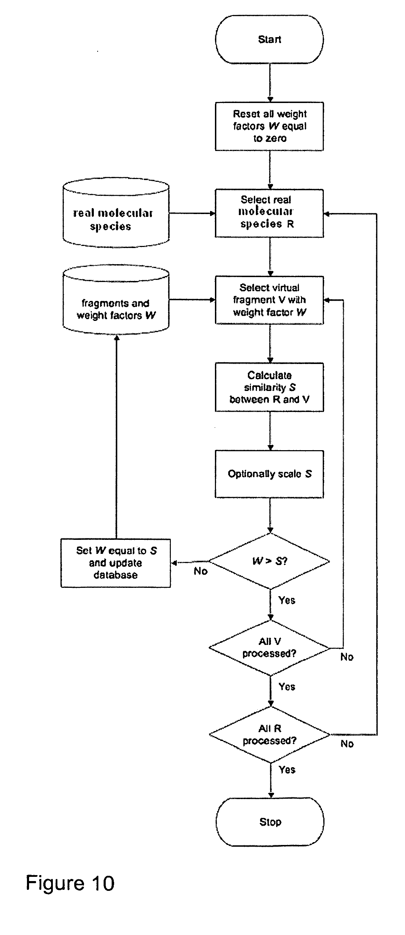

The conversion from the experimental binding data to weight factors is achieved by the calculation of chemical similarities between each of the 'real molecular species' on the one hand and the fragment entries in the fragment database on the other hand. The process to achieve this is illustrated in Figure 10 and includes the following:

1. Reset the weight factors $W$ of all the database fragments to zero.
2. Select a real molecular species which needs to be included and call it R.
3. Loop over all the fragment entries in the fragment database and calculate for each fragment V the topological similarity with a representation of R. Set this similarity value equal to $S$. A multitude of similarity measurement methods may be used. Preferably, the topology similarity based on the Tanimoto index is used. The similarity measurement method should quantify low similarities as values being close to zero, and large similarities as values being close to one.
4. The calculated similarity $S$ may optionally be scaled by some measure of the experimental binding affinity of the real molecular species, if available. For example, a typical scaling factor could be the log-transform of the negative $IC_{50}$-value of the particular real molecular species. Applying such a scaling factor will lead to prioritization of the virtual fragments being similar to the high affinity real molecular species, and will discriminate against the virtual fragments that are more similar to the low affinity real molecular species.
5. If the calculated similarity $S$ is larger than the current weight factor $W$ of a particular fragment in the fragment database, the weight factor $W$ is replaced by the calculated similarity value $S$.
6. Repeat from step 2 until a chosen number, preferably all real molecular species have been selected.

According to the here described procedure, all database fragments with a high similarity with at least one of the selected real molecular species will get a high weight factor assigned to it, and therefore these virtual fragments will be more likely to be selected during the synthesis phase.

Transformation of the weight factors

Once initial weight factors have been generated from, for instance, the available binding affinities, these weight factors may be further modified by multiplication with a factor *f*:

$$w_n = w_i f \qquad \text{(Equation 1)}$$

in which $w_n$ stands for the transformed weight factor, and $w_i$ the initial weight factor as derived from, for instance, experimental binding data.

The transformation factor *f* can be generated from a multitude of sources, including but not limited to the following examples:

- The frequency of occurrence of the particular fragment in an ensemble of represented existing molecules. The inclusion of information on the frequency of occurrence leads to a more likely selection of fragments that are more common in the ensemble of represented existing molecules, and therefore more likely in terms of synthetic accessibility. For example, the CO<20> fragment has in the database of example 1 below a frequency of 0.13, which means that 13% of all represented existing molecules in this database contains this fragment. The C(<1 >)O<20> linker has a relative frequency of 0.02 in this same database, which means that 2% of all represented existing molecules of this database contain this particular linker.
- Physicochemical property data may also be used as the transformation factor *f*, such as for example the number of atoms in each fragment, the inverse of the logP (P being the partition coefficient), the number of rings, and many more properties. The advantage of this approach is that the synthesis phase can be steered into the direction the user defines by modifying the weight factors accordingly. For example, guiding the synthesis phase towards the generation of virtual molecules with a low logP is achieved by defining weight factors that are related to the inverse of the fragment logP.
- A constant number, for example f being equal to 0.1.
- A mathematical conversion, such as for example the logarithm or the inverse of the transformation factor.
- A multiplication or a summation of two or more of the above mentioned transformation factors, such as in equations 2 and 3:

$$f = \prod_{i=1}^{n} f_i \qquad \text{(Equation 2)}$$

$$f = \sum_{i=1}^{n} f_i \qquad \text{(Equation 3)}$$

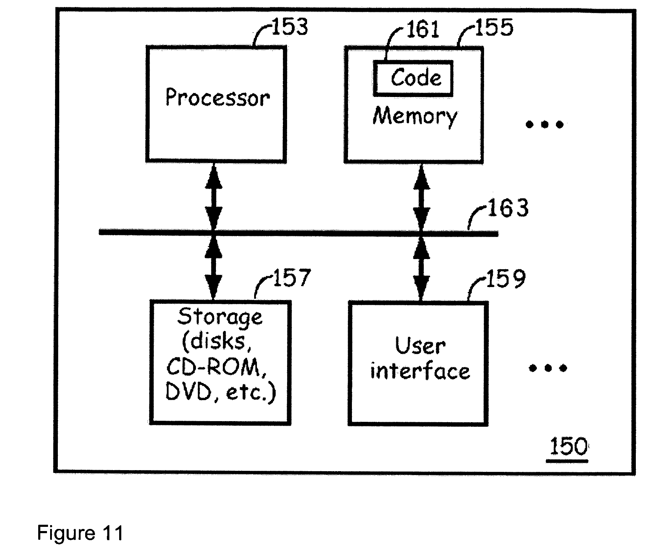

Such method embodiments as are described above may be implemented in a processing system 150 such as shown in FIG. 11. FIG. 11 shows one configuration of processing system 150 that includes at least one programmable processor 153 coupled to a memory subsystem 155 that includes at least one form of memory, e.g., RAM, ROM, and so forth. A storage subsystem 157 may be included that has at least one disk drive and/or CD-ROM drive and/or DVD drive. In some implementations, a display system, a keyboard, and a pointing device may be included as part of a user interface subsystem 159 to provide for a user to manually input information. Ports for inputting and outputting data also may be included. More elements such as network connections, interfaces to various devices, and so forth, may be included, but are not illustrated in FIG. 11. The various elements of the processing system 150 may be coupled in various ways, including via a bus subsystem 163 shown in FIG. 11 for simplicity as a single bus, but will be understood to those in the art to include a system of at least one bus. The memory of the memory subsystem 155 may at some time hold part or all (in either case shown as 161) of a set of instructions that when executed on the processing system 150 implement the step(s) of any of the method embodiments described herein. Thus, while a processing system 150 such as shown in FIG. 11 is prior art, a system that includes the instructions to implement novel aspects of the present invention is not prior art, and therefore FIG. 11 is not labelled as prior art.

It is to be noted that the processor 153 or processors may be a general purpose, or a special purpose processor, and may be for inclusion in a device, e.g., a chip that has other components that perform other functions, for example it may be an embedded processor. Also with developments such devices may be replaced by any other suitable processing engine, e.g. an FPGA. Thus, one or more aspects of the present invention can be implemented in digital electronic circuitry, or in computer hardware, firmware, software, or in combinations of them. Method steps of aspects of the invention may be performed by a programmable processor executing instructions to perform functions of those aspects of the invention, e.g., by operating on input data and generating output data.

Furthermore, aspects of the invention can be implemented in a computer program product tangibly embodied in a carrier medium carrying machine-readable code for execution by a programmable processor. The term "carrier medium" refers to any medium that participates in providing instructions to a processor for execution. Such a medium may take many forms, including but not limited to, non-volatile media, and transmission media. Non-volatile media includes, for example, optical or magnetic disks, such as a storage device which is part of mass storage. Volatile media includes mass storage. Volatile media includes dynamic memory such as RAM. Common forms of computer readable media include, for example a floppy disk, a flexible disk, a hard disk, magnetic tape, or any other magnetic medium, a CD-ROM, any other optical medium, punch cards, paper tapes, any other physical medium with patterns of holes, a RAM, a PROM, an EPROM, a FLASH-EPROM, any other memory chip or cartridge, a carrier wave as described hereafter, or any other medium from which a computer can read. Various forms of computer readable media may be involved in carrying one or more sequences of one or more instructions to a processor for execution. For example, the instructions may initially be carried on a magnetic disk of a remote computer. The remote computer can load the instructions into its dynamic memory and send the instructions over a telephone line using a modem. A modem local to the computer system can receive the data on the telephone line and use an infrared transmitter to convert the data to an infrared signal. An infrared detector coupled to a bus can receive the data carried in the infrared signal and place the data on the bus. The bus carries data to main memory, from which a processor retrieves and executes the instructions. The instructions received by main memory may optionally be stored on a storage device either before or after execution by a processor. The instructions can also be transmitted via a carrier wave in a network, such as a LAN, a WAN or the Internet. Transmission media can take the form of acoustic or light waves, such as those generated during radio wave and infrared data communications. Transmission media include coaxial cables, copper wire and fibre optics, including the wires that comprise a bus within a computer.

Example 1: building and cleaning of a database of representations of existing molecules.

A large number of represented existing molecules have been collected from the library sources or vendors listed in the table below and an original database comprising in total more than 7 million original molecules has been build.

| Library source or vendor | Number of original molecules | molecules remaining after cleaning step |
|---|---|---|
| ACB Blocks | 1,280 | 648 |
| Akos | 230,148 | 74,889 |
| Ambinter | 1,360,060 | 401,122 |
| A Synthese Biotech | 16,122 | 0 |
| AstaTech | 2,224 | 549 |
| Asinex | 372,703 | 127,963 |
| Aurora Feinchemie | 1,019,555 | 305,231 |
| ChemBridge | 482,993 | 200,666 |
| ChemDiv | 605,230 | 207,539 |
| Cerep | 29,231 | 10,179 |
| CMC | 8,757 | 2,657 |
| ChemStar | 60,260 | 15,128 |
| Chem T&I | 323,127 | 87,111 |
| Enamine | 467,645 | 160,129 |
| Exclusive Chemistry | 1,906 | 961 |
| InterBioScreen | 378,553 | 104,891 |

(continued)

| Library source or vendor | Number of original molecules | molecules remaining after cleaning step |
|---|---|---|
| KeyOrganics | 47,632 | 17,770 |
| Life Chemicals | 277,347 | 104,136 |
| Matrix Scientific | 15,183 | 5,323 |
| Maybridge | 81,077 | 27,245 |
| MDPI | 10,655 | 2,522 |
| Menai Organics | 77 | 17 |
| Microsource | 2,000 | 580 |
| Nanosyn | 65,328 | 19,596 |
| Analyticon Discovery | 8,801 | 2,234 |
| NCI | 250,251 | 16,998 |
| Otava | 139,116 | 45,146 |
| Pharmeks | 156,535 | 34,497 |
| Peakdale | 8,548 | 4,164 |
| Prestwick | 1,375 | 474 |
| Sigma Aldrich | 205,823 | 30,826 |
| Specs | 203,922 | 64,712 |
| Synchem | 1,542 | 208 |
| Tocris | 979 | 349 |
| TimTec | 669,701 | 188,538 |
| TOSlab | 15,941 | 3,089 |
| Vitas-M Laboratory | 224,289 | 67,071 |
| Zerenex | 39,805 | 12,726 |
| Total | 7,785,721 | 2,347,884 |

After a cleaning procedure consisting in removing from the database the following molecules:

- Molecules containing an atom which is different from the following: H, C, N, O, F, S, Cl, Br, I.
- Molecules containing a functional group which is one of the following:

quinone; pentafluorophenyl esters; paranitrophenyl esters; triflates; lawesson-s-reagent; phosphoramides; acylhydrazide; cation C, Cl, I, P, or S; phosphoryl; alkyl phosphate; phosphinic acid; phosphanes; phosphoranes; chloramidines; nitroso; N-, P-, S-halides; carbodiimide; isonitrile; triacyloxime; cyanohydrins; acylcyanides; sulfonylnitrile; phosphonylnitrile; azocyanamides; beta-azocarbonyl; polyenes; saponin derivatives; acid halide; aldehyde; alkylhalide; anhydride; azide; azo; dipeptide; michael acceptor; betahalocarbonyl; nitro; oxygen cation; peroxide; phosphonic acid; phosphonic ester; phosphoric acid; phosphoric ester; sulfonic acid; sulfonic ester; tricarbophosphene; epoxide; sulfonylhalide; halopyrimidine; perhalo-ketone; aziridine; alphahalo-amine; halo-amine; halo-alkene; acyclic NCN; acyclic NS; $SCN_2$; terminal vinyl; hydrazine; N-methoyl; NS-betahalothyl; propiolactones; nitroso; iodoso; iodoxy; N-oxide; iodine; phosphonamide; alphahalo ketone; oxaziridine; sulfonimine; sulfinimine; phosphoryl; sulfinylthio; disulfide; enol ether; enamine; organometallic; dithioacetal; isothiocyanate; isocyanate; carbamic acid; triazine; nonacylhydrazone; thiourea; hemiketal; hemiacetal; ketal; aminal; hemi-aminal; benzyloxycarbonyl; tert-buthoxycarbonyl; fluorenylmethoxycarbonyl; trimethylsilyl; tert-butyldimethylsilyl; triisopropylsilyl; tert-butyldiphenylsilyl,

The final cleaned database comprises approximately 2.3 million entries.

Example 2: de novo generation of new molecules with similar shape as that of a reference molecule

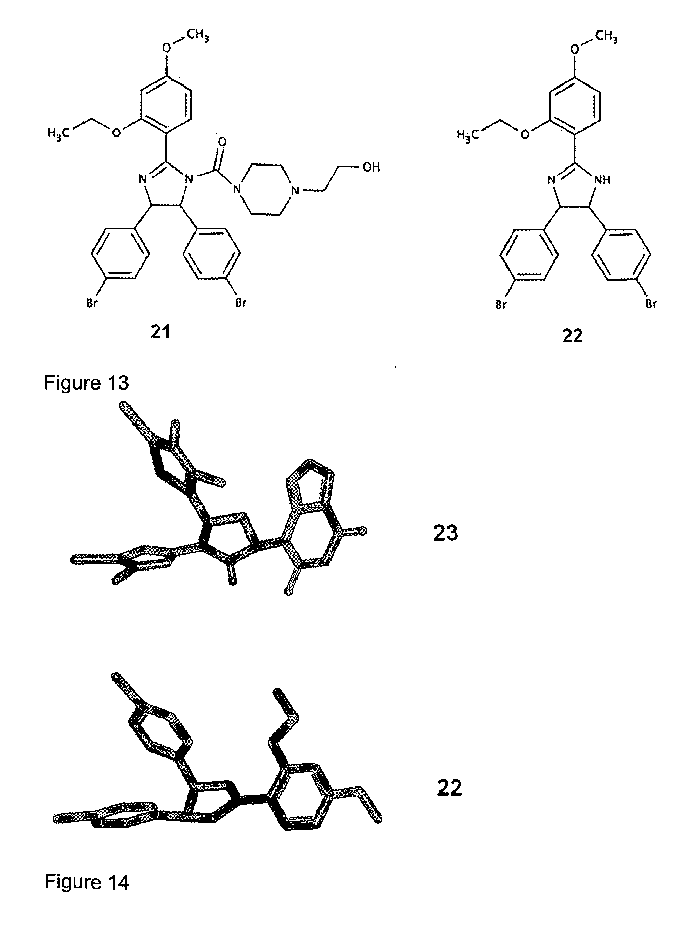

A modified Nutlin-2 molecule was used as reference (22) (see Figure 13). Nutlin-2 (21) has been described as being a potent inhibitor of the MDM2/p53 protein-protein interaction [Vassilev, L.T. (2005) J. Med. Chem. 48, 4491-4499]. For the purpose of this example, the reference Nutlin-2 molecule (22) was structurally modified so that only the functional moieties of the molecule which have been described to be involved in the binding to the MDM2 protein were retained, while all the non-binding atoms of the Nutlin-2 were removed from the molecule. The structures of both the original Nutlin-2 (21) and the modified version (22) which is used as a reference molecule in this example, are shown in Figure 13.

The conformation of the reference molecule was obtained from the X-ray structure of Nutlin-2 in complex with human MDM2 [Vassilev, L.T. (2004) Science 303, 844-848]. The coordinates of the Nutlin-2 atoms were transferred to the corresponding atoms of the reference molecule.

Novel molecules were generated using the genetic algorithm approach as described in this patent. The fragment (13) and connectivity (14) database were generated from analysing all 2.3 million compounds from example 1 using the atom labelling scheme of Table 1. The resulting fragment database (13) contained a total of 85,481 fragments having one or more open bonds, and the resulting connectivity rules database (14) was made up of 147 connectivity rules. Of these 147 connectivities, 15 consisted of a double bond order. The total population used in the genetic algorithm consisted of 250 molecules during the entire run, a crossover ratio of 0.1 and a mutation ratio of 0.3 was used.

The fitness scores of the population molecules were derived by calculating the shape similarity between the individual population molecules and the reference molecule. For each of the molecules, this resulted in a fitness score between 0 and 1, whereby larger numbers are indicative of a better shape similarity between the reference and the molecule under consideration. In order to calculate the fitness scores, a combination of the OMEGA and ROCS programs from OpenEye Scientific Software Inc was used. For this particular example, OMEGA was used to generate in first instance a number of conformations of each of the individual population molecules, and ROCS was subsequently used to calculate for each of the population molecules the shape similarity between all the conformations of the particular molecule and the reference molecule. From all conformations of each molecule, the highest shape similarity was taken as being the fitness score for the particular molecule. The particular program settings were as follows:

- To generate the multi-conformations of each molecule, OMEGA version 2.0 was used. A maximum of 100 conformations were generated of each molecule, with an energy window cutoff of 5.0 kcal/mol above the lowest energy conformation.
- To generate the shape similarity with the reference compound, ROCS version 2.2 was used. The 'ImplicitMillsDean' coloring scheme was used to ensure that not only the shape, but also atom type matching, was taken into account for the calculation of the similarity Tanimoto coefficient. This coefficient was generated by the ROCS program as the 'ComboScore', which is a number between 0 (no similarity) and 2 (highest similarity). This number was then dived by 2 to convert the range of similarity measures between 0 and 1.

All molecules that failed the OMEGA/ROCS programs were given a fitness score of zero.

The entire process was run for 1,000 cycles. After this period, the fitness score of the best molecule (23) from the entire population was 0.7775. A comparison of the best molecule (23) and the reference molecule (22) is shown in Figure 14.

**Claims**

1. A computer-based method of evolving a virtual molecule with a set of desired properties for binding at a target molecule comprising the steps of:

    a) storing *in silico* labelled fragments of represented existing molecules in one or more fragment databases, said one or more fragment databases being machine readable by a computer system, said labelled fragments having one or more open connections, said labelled fragments being obtainable by:

    (i) *in silico* labelling chosen atoms of a set of represented existing molecules with labels, each label giving information relative to both the chemical nature and the connectivity of said atoms, and
    (ii) cutting said represented existing molecules into one or more labelled fragments,

    b) determining which label is connected to which label in each of said represented existing molecules and storing this information as connectivity rules in a connectivity database,
    c) generating one or more virtual molecules by combining at least two of said labelled fragments from said one or more fragments database by matching said labels according to said connectivity rules from said connectivity database,

d) determining the degree of fitness of each said virtual molecules against a fitness function,

e) selecting one or more times at least one virtual molecule correlating positively with the degree of fitness,

f) modifying each of said at least one selected virtual molecule,

g) repeating iteratively steps (d) to (f) until either:

1) the degree of fitness of at least one of said virtual molecules selected in (e) is equal or higher than a predefined target degree of fitness or

2) step (e) has been performed a predefined number of times,

wherein once (1) or (2) is achieved, step (h) is performed instead of step (f),

h) generating a data file comprising electronic data representing said at least one virtual molecule selected in (g).

2. A computer-based method according to claim 1, wherein said data file further comprises electronic data representing the degree of fitness of said at least one virtual molecule or another value correlating with said degree of fitness.

3. A computer-based method according to claim 2, wherein said other value is a predicted biological activity.

4. A computer-based method according to claim 3, wherein said predicted biological activity is a binding affinity to said target molecule.

5. A computer-based method according to any of claims 1 to 4, wherein said labelled fragments having one open connection are stored into a first fragment database and wherein said labelled fragments having two or more open connections are stored in a second fragment database.

6. A computer-based method according to any of claims 1 to 5, wherein the number of virtual molecules generated in step (c) is from 50 to 1000.

7. A computer-based method according to any of claims 1 to 6, wherein in step (c) said at least two labelled fragments are selected randomly in the one or more fragment database.

8. A computer-based method according to any of claims 1 to 7, wherein each of said labelled fragments is associated to a weight factor and wherein in step (c) said at least two labelled fragments are selected correlating positively with said weight factor.

9. A computer based method according to any of claims 1 to 8, wherein said chosen atoms are all atoms but hydrogen atoms.

10. A computer-based method according to claim 8, wherein said weight factor correlates positively with an experimentally determined binding affinity between real molecular species and said target molecule wherein said real molecular species are structurally related to said labelled fragment to which said weight factor is associated.

11. A computer-based method according to claim 10, wherein said real molecular species have a molecular weight smaller or equal to 350 g/mol.

12. A computer-based method according to any of claim 10 or claim 11, wherein said binding affinity is determined via one of the following technique: X-ray crystallography, NMR, mass spectrometry, microcalorimetry, solid-phase detection, in vitro binding assay, sedimentation analysis or capillary electrophoresis.

13. A computer-based method according to any of claim 10 to 12, wherein said binding affinity is binary.

14. A computer-based method according to any of claims 8 to 13, wherein said weight factor correlates positively with the frequency of its associated labelled fragment in said one or more fragment databases.

15. A computer-based method according to any of claims 1 to 14, wherein said existing molecules are stable under normal physiological conditions.

**16.** A computer program product comprising software code for implementing any of the methods of the claims 1 to 15 when executed on a computing system.

**17.** A machine readable data carrier storing the computer program of claim 16.

**18.** A method of manufacturing a molecule using the method of any of the claims 1 to 15.

Figure 1

Figure 2

Figure 3

Figure 4

Figure 5

Figure 6

Figure 7

Figure 8

Figure 9

Figure 10

Figure 11

Figure 12

**21**

**22**

Figure 13

**23**

**22**

Figure 14

European Patent
Office

**EUROPEAN SEARCH REPORT**

Application Number

EP 06 01 8169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FECHNER ULI ET AL: "Flux (1): a virtual synthesis scheme for fragment-based de novo design." JOURNAL OF CHEMICAL INFORMATION AND MODELING 2006 MAR-APR, vol. 46, no. 2, March 2006 (2006-03), pages 699-707, XP002435751 ISSN: 1549-9596 * page 699, right-hand column, line 35 - page 704, left-hand column, line 34 * * figures 1-4 * | 1-7,9, 15-18 | INV. G06F19/00 |
| A | PIROK GYÖRGY ET AL: "Making "real" molecules in virtual space." JOURNAL OF CHEMICAL INFORMATION AND MODELING 2006 MAR-APR, vol. 46, no. 2, March 2006 (2006-03), pages 563-568, XP002435752 ISSN: 1549-9596 * abstract * * page 563, right-hand column, line 4 - page 565, left-hand column, line 20 * | 1-18 | |
| A | VINKERS H M ET AL: "SYNOPSIS: SYNthesize and OPtimize System in Silico" JOURNAL OF MEDICINAL CHEMISTRY, AMERICAN CHEMICAL SOCIETY. WASHINGTON, US, vol. 46, 2003, pages 2765-2773, XP002259660 ISSN: 0022-2623 * page 2766, left-hand column, line 37 - page 2768, right-hand column, line 4 * | 1-18 | TECHNICAL FIELDS SEARCHED (IPC) G06F |

-/--

The present search report has been drawn up for all claims

| Place of search | Date of completion of the search | Examiner |
|---|---|---|
| The Hague | 31 May 2007 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&: member of the same patent family, corresponding document

EPO FORM 1503 03.82 (P04C01)

**EP 1 895 436 A1**

**European Patent Office**

**EUROPEAN SEARCH REPORT**

Application Number

EP 06 01 8169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PEGG S C-H ET AL: "A genetic algorithm for structure-based de novo design" JOURNAL OF COMPUTER-AIDED MOLECULAR DESIGN, ESCOM SCIENCE PUBLISHERS BV, vol. 15, no. 10, October 2001 (2001-10), pages 911-933, XP002318551 ISSN: 0920-654X * page 912, right-hand column, line 10 - page 915, right-hand column, line 38 * * figures 1,3 * | 1-18 | |
| A | SCHNEIDER GISBERT ET AL: "Computer-based de novo design of drug-like molecules." NATURE REVIEWS. DRUG DISCOVERY AUG 2005, vol. 4, no. 8, August 2005 (2005-08), pages 649-663, XP002435753 ISSN: 1474-1776 * page 655, right-hand column, line 16 - page 656, left-hand column, line 54 * * page 656, right-hand column, line 54 - page 657, right-hand column, line 44 * * page 659, left-hand column, line 6 - right-hand column, line 40 * * page 660, right-hand column, lines 40-46 * | 1-18 | TECHNICAL FIELDS SEARCHED (IPC) |
| A | SCHNEIDER G ET AL: "DE NOVO DESIGN OF MOLECULAR ARCHITECTURES BY EVOLUTIONARY ASSEMBLY OF DRUG-DERIVED BUILDING BLOCKS" JOURNAL OF COMPUTER-AIDED MOLECULAR DESIGN, ESCOM SCIENCE PUBLISHERS BV, vol. 14, no. 5, 2000, pages 487-494, XP009025686 ISSN: 0920-654X * abstract * | 1-18 | |

-/--

The present search report has been drawn up for all claims

| Place of search | Date of completion of the search | Examiner |
|---|---|---|
| The Hague | 31 May 2007 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**European Patent Office**

| | Application Number |
|---|---|
| | EP 06 01 8169 |

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WESTHEAD D R ET AL: "PRO_LIGAND: AN APPROACH TO DE NOVO MOLECULAR DESIGN. 3. A GENETIC ALGORITHM FOR STRUCTURE REFINEMENT" JOURNAL OF COMPUTER-AIDED MOLECULAR DESIGN, ESCOM SCIENCE PUBLISHERS BV, vol. 9, 1995, pages 139-148, XP008043058 ISSN: 0920-654X * abstract * * page 140, left-hand column, lines 20-23 * ----- | 1-18 | |
| | | | TECHNICAL FIELDS SEARCHED (IPC) |

The present search report has been drawn up for all claims

| Place of search | Date of completion of the search | Examiner |
|---|---|---|
| The Hague | 31 May 2007 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

### Patent documents cited in the description

- US 5434796 A

### Non-patent literature cited in the description

- **CAFLISCH et al.** *J. Med. Chem.,* 1993, vol. 36, 2142-2167
- **NISHIBATA et al.** *J. Med. Chem.,* 1993, vol. 36, 2921-2928
- **GILLET et al.** *J. Chem. Inf. Comput. Sci.,* 1994, vol. 34, 207-217
- **BRADLEY.** *Horizon Symposium Nature,* 2004
- **DICKSON ; GAGNON.** *Nat. Rev. Drug Discov.,* 2004, vol. 3, 417-429
- **SERVICE.** *Science,* 2004, vol. 303, 1796-1799
- **HANN ; GREEN.** *Curr. Opin. Chem. Biol.,* 1999, vol. 3, 379-383
- **LOBANOV ; AGRAFIOTIS.** *Comb. Chem. High Throughput Screen.,* 2002, vol. 5, 167-178
- **LEACH ; HANN.** *Drug Discov. Today,* 2000, vol. 5, 326-336
- **LELAND et al.** *J. Chem. Inf. Comput. Sci.,* 1997, vol. 37, 62-70
- **AGRAFIOTIS.** *J. Comput. Aided Mol. Des.,* 2002, vol. 16, 335-356
- **AGRAFIOTIS ; LOBANOV.** *J. Chem. Inf. Comput. Sci.,* 2000, vol. 40, 1030-1038
- **WEININGER.** *J. Chem. Inf. Comput. Sci.,* 1988, vol. 28, 31-36
- **KAMPHAUSEN et al.** *J. Comput. Aided Mol. Des.,* 2002, vol. 16, 551-567
- **VENKATASUBRAMANIAN et al.** *J. Chem. Inf. Comput. Sci.,* 1995, vol. 35, 188-195
- Computational Medicinal Chemistry for Drug Discovery. Marcel Dekker
- **BROWN et al.** *J. Chem. Inf. Comput. Sci.,* 2004, vol. 44, 1079-1087
- **GLOBUS et al.** *Nanotechnology,* 1999, vol. 10, 290-299
- **NACHBAR.** *Genetic Programming and Evolvable Machines,* 2000, vol. 1, 57-94
- **BEMIS ; MURCKO.** *J. Med. Chem.,* 1996, vol. 39, 2887-2893
- **EWING et al.** *J. Comput. Aided Mol. Des.,* 2001, vol. 15, 411-428
- **RAREY et al.** *J. Mol. Biol.,* 1996, vol. 261, 470-489
- **HALGREN et al.** *J. Med. Chem.,* 2004, vol. 47, 1750-1759
- **JONES et al.** *J. Mol. Biol.,* 1997, vol. 267, 727-748
- **PEROLA et al.** *Proteins,* 2004, vol. 56, 235-249
- **BARNARD.** *J. Chem. Inf. Comput. Sci.,* 1993, vol. 33, 532-538
- **KLEBE et al.** *J. Comput. Aided Mol. Des.,* 1999, vol. 13, 35-49
- **LEMMEN ; LENGAUER.** *J. Comput. Aided Mol. Des.,* 2000, vol. 14, 215-232
- **GRANT et al.** *J. Comp. Chem.,* 1996, vol. 17, 1653-1666
- **SHERIDAN et al.** *Proc. Natl. Acad. Sci. USA.,* 1989, vol. 86, 8165-8169
- **GILLET.** *Methods Mol. Biol.,* 2004, vol. 275, 335-354
- **VALLER ; GREEN.** *Drug Discov. Today,* 2000, vol. 5, 286-293
- **ZHENG.** *Methods Mol. Biol.,* 2004, vol. 275, 379-398
- **YOUNG et al.** *J. Chem. Inf. Comput. Sci.,* 2003, vol. 43, 1916-1921
- **MILLER et al.** J. Chem. Inf. Comput introduced an approach based on information theory. *Sci.,* 2003, vol. 43
- **SCHNEIDER ; NETTEKOVEN.** *J. Comb. Chem.,* 2003, vol. 5, 233-237
- **GILLET et al.** *J. Chem. Inf. Comput. Sci.,* 1999, vol. 39, 169-177
- **GILLET et al.** *J. Chem. Inf. Comput. Sci.,* 2002, vol. 42, 375-385
- **HALGREN.** *J. Comp. Chem.,* 1996, vol. 17, 490-519
- **HALGREN.** *J. Comp. Chem.,* 1996, vol. 17, 520-552
- **HALGREN.** *J. Comp. Chem.,* 1996, vol. 17, 553-586
- **HALGREN.** *J. Comp. Chem.,* 1996, vol. 17, 616-641
- **HALGREN.** *J. Comp. Chem.,* 1999, vol. 20, 720-729

- **HALGREN.** *J. Comp. Chem.,* 1999, vol. 20, 730-748
- **HALGREN ; NACHBAR.** *J. Comp. Chem.,* 1996, vol. 17, 587-615
- **BALDUCCI ; PEARLMAN.** *J. Chem. Inf. Comput. Sci.,* 1994, vol. 34, 822-831
- **MORRIS et al.** *J. Comp. Chem.,* 1998, vol. 19, 1639-1662
- **BAKER.** Proceedings of the Second International Conference on Genetic Algorithms and their Application. Lawrence Erlbaum Associates, 1987, 14-21
- **GOLDBERG.** Genetic algorithms in search, optimisation, and machine learning. Addison-Wesley, 1989
- **JAHNKE ; ERLANSON ; MANNHOLD ; KUBINYI ; FOLKERS.** Fragment-based approaches in drug discovery. Wiley, 2006
- **REES et al.** *Nature Rev. Drug Discov.,* 2004, vol. 3, 660-672
- **HARTSHORN et al.** *J. Med. Chem.,* 2005, vol. 48, 403-413
- **SHUKER et al.** *Science,* 1996, vol. 274, 1531-1534
- **DELANO.** *Curr. Opin. Struct. Biol.,* 2002, vol. 12, 14-20
- **SWAYZE et al.** *J. Med. Chem.,* 2002, vol. 45, 3816-3819
- Divided we fall? Studying low affinity real molecular species of ligands by ITS. *MicroCal LLC,* 2005
- **BOEHM et al.** *J. Med. Chem.,* 2000, vol. 43, 2664-2674
- **LEBOWITZ et al.** *Protein Sci.,* 2002, vol. 11, 2067-2079
- **CARBECK et al.** *Acc. Chem. Res.,* 1998, vol. 31, 343-350
- **VASSILEV, L.T.** *J. Med. Chem.,* 2005, vol. 48, 4491-4499
- **VASSILEV, L.T.** *Science,* 2004, vol. 303, 844-848